# EUROPÄISCHE PATENTSCHRIFT

(11) **EP 3 277 657 B1**
(45) Veröffentlichungstag und Bekanntmachung des Hinweises auf die Patenterteilung: **15.05.2019**
(21) Anmeldenummer: 16712342.1
(22) Anmeldetag: 30.03.2016
(51) Int. Cl.: C07C 67/04, C07C 69/54

(54) **HERSTELLUNG VON TERT-BUTYLESTERN ALIPHATISCHER CARBONSÄUREN**
PRODUCTION OF TERT-BUTYL ESTERS OF ALIPHATIC CARBOXYLIC ACIDS
PRODUCTION DE TERT-BUTYLESTERS D'ACIDES CARBOXYLIQUES ALIPHATIQUES

(30) Priorität: 31.03.2015 DE 102015205752; 31.03.2015 US 201562140473 P
(43) Veröffentlichungstag der Anmeldung: 07.02.2018
(73) Patentinhaber: BASF SE, 67056 Ludwigshafen am Rhein (DE)
(72) Erfinder: HORSTMANN, Catharina, 68549 Ilvesheim (DE); HECHLER, Claus, 67063 Ludwigshafen (DE); GRACKIEWICZ, Gregor, 67063 Ludwigshafen (DE); SCHALL, Bernd, 67575 Eich (DE)
(74) Vertreter: BASF IP Association
(86) Internationale Anmeldenummer: PCT/EP2016/056936
(87) Internationale Veröffentlichungsnummer: WO 2016/156410

(56) Entgegenhaltungen:
- EP-A1- 1 097 742
- EP-A1- 2 017 255
- WO-A2-02/10110

## Beschreibung

Die vorliegende Erfindung betrifft ein kontinuierliches Verfahren zur Herstellung von tert-Butylestern aliphatischer C₁-C₄-Carbonsäure durch Umsetzung der Carbonsäure mit Isobuten.

Die tert-Butylester aliphatischer C₁-C₄-Carbonsäuren finden vielfältige Anwendung. Die tert-Butylester gesättigter aliphatischen Carbonsäuren wie tert-Butylacetat sind beispielsweise begehrte Lösungsmittel. (Meth)acrylsäure-tert-butylester sind wichtige Ausgangsstoffe zur Herstellung von Polymerisaten, die u. a. als Bestandteil von Anstrichmitteln, Klebstoffen oder Lackharzen Anwendung finden. Die Herstellung von tert-Butylestern erfolgt im Allgemeinen durch säurekatalysierte Addition einer Carbonsäure an Isobuten (Houben-Weyl, Methoden der Organischen Chemie, Bd. 8, 1952, S. 534; US 3,031,495 und US 3,082,246). Als Katalysatoren verwendet man dabei im Reaktionsgemisch lösliche Säuren, z. B. Mineralsäuren oder Alkyl- bzw. Arylsulfonsäuren (DE-A-12 49 857, US 3,087,962, US 3,088,969) oder unlösliche Katalysatoren wie saure Austauscherharze (US 3,037,052, US 3,031,495, DE-A-31 05 399, EP-A-268 999).

Die WO 02/10109 beschreibt ein Verfahren zur kontinuierlichen Herstellung des tert-Butylesters einer aliphatischen C₁-C₄-Carbonsäure durch Umsetzung der Carbonsäure mit Isobuten in flüssiger Phase in Gegenwart eines sauren Katalysators, wobei man die Umsetzung in einem in mehrere Abschnitte unterteilten Reaktor durchführt, die Carbonsäure, das Isoolefin und den Katalysator in den ersten Abschnitt des Reaktors einspeist, das erhaltene Reaktionsgemisch aus dem letzten Abschnitt des Reaktors entnimmt und den Ester daraus gewinnt, wobei man die Reaktionstemperatur im Reaktor so kontrolliert, dass sie im Bereich von 10 bis 40 °C liegt und im ersten Abschnitt des Reaktors am höchsten ist.

Die WO 02/10110 beschreibt ein Verfahren zur Herstellung eines tert-Alkyl-(meth)acrylats durch Umsetzung von (Meth)acrylsäure mit einem Olefin in homogener Phase in Gegenwart eines sauren Katalysators und Gewinnung des tert-Alkyl-(meth)acrylats aus dem Reaktionsgemisch, wobei man den Katalysator durch eine zweistufige Destillation des Reaktionsgemisches als Rückstand abtrennt und das tert-Alkyl (meth) acrylat aus den Destillaten gewinnt. Die WO 02/10110 beschreibt eine Kondensation der Brüden in zwei in Serie geschalteten Kondensatoren, wobei der zweite Kondensator mit niedrigerer Kühltemperatur betrieben wird.

Der Umsatz der Carbonsäure mit Isobuten verläuft in der Regel nicht quantitativ. Es ist daher wünschenswert, nicht umgesetztes Isobuten auf einfache Weise möglichst weitgehend aus dem Veresterungsgemisch abzutrennen und zurückzuführen. Ein Problem der bekannten Verfahren ist die Tatsache, dass die Abtrennung des nicht umgesetzten Isobutens durch Partialkondensation des bei der Katalysatorabtrennung anfallenden Brüdens aufgrund der Löslichkeit des Isobutens im tert-Butylester mit ungenügender Trennschärfe erfolgt. Dies hat zur Folge, dass mit dem Isobuten-Kreisgas auch tert-Butylester wieder in den Synthesereaktor zurückgeführt wird und/oder signifikante Mengen Isobuten erst im Zuge der weiteren Aufarbeitung bei der Reindestillation des tert-Butylesters abgetrennt werden.

Der vorliegenden Erfindung liegt daher die Aufgabe zugrunde, ein Verfahren zur Herstellung des tert-Butylesters einer aliphatischen C₁-C₄-Carbonsäure durch Umsetzung der Carbonsäure mit Isobuten bereitzustellen, bei dem nicht umgesetztes Isobuten energetisch günstig und mit einem verbesserten Abtrennungsgrad aus dem Veresterungsgemisch isoliert wird.

Die Aufgabe wird gelöst durch ein Verfahren zur kontinuierlichen Herstellung des tert-Butylesters einer aliphatischen C₁-C₄-Carbonsäure, bei dem man:
a) eine aliphatische C₁-C₄-Carbonsäure mit Isobuten in Gegenwart eines sauren Katalysators zu einem Veresterungsgemisch (G1) umsetzt;
b) das Veresterungsgemisch (G1) partiell verdampft, wodurch man eine den sauren Katalysator enthaltende flüssige erste Schwersiederphase (SPh1), und einen tert-Butylester enthaltenden ersten Brüden (B1) erhält;
c) den ersten Brüden (B1) fraktionierend kondensiert, indem man den ersten Brüden (B1) bei einem ersten Druck und einer ersten Temperatur partiell kondensiert und ein erstes Kondensat (K1) erhält, den nicht kondensierten zweiten Brüden (B2) bei einem zweiten Druck und einer zweiten Temperatur partiell kondensiert und ein zweites Kondensat (K2) erhält, wobei die erste Temperatur 0 bis 45 °C unterhalb der Kondensationstemperatur des tert-Butylesters beim ersten Druck liegt und die zweite Temperatur 45 bis 80 °C unterhalb der Kondensationstemperatur des tert-Butylesters beim zweiten Druck liegt, mit der Maßgabe, dass die zweite Temperatur mindestens 5 °C unterhalb der ersten Temperatur liegt; und
d) das erste Kondensat (K1) und das zweite Kondensat (K2) vereinigt und einer gemeinsamen Aufarbeitung zuführt, und den bei der zweiten Temperatur nicht kondensierten dritten Brüden (B3) in den Schritt a) zurückführt.

Als Kondensationstemperatur einer Verbindung wird die Temperatur bezeichnet, ab der die Verbindung bei einem gegebenen Druck kondensiert, das heißt vom gasförmigen in den flüssigen Aggregatzustand übergeht. Unter einer partiellen Kondensation wird eine unvollständige Kondensation verstanden, insbesondere eine Kondensation, bei der neben den permanenten Gasen auch ein Teil der organischen Verbindungen in der Gasphase verbleibt. Die hier angegebenen Temperaturen der partiellen Kondensationen beziehen sich auf die Temperatur des Kondensats bei Entnahme aus dem jeweiligen Kondensator.

Bei der ersten Kondensation bei einer Temperatur bei oder knapp unter der Kondensationstemperatur des tert-Butylesters wird eine Hauptmenge des tert-Butylesters auskondensiert, während verhältnismäßig wenig Isobuten co-kondensiert. Der nicht kondensierte Brüden wird bei einer tieferen zweiten Temperatur partiell kondensiert. Bei der zweiten partiellen Kondensation wird anteilig eine größere Menge an Isobuten cokondensiert; allerdings ist der Volumenstrom in der Regel deutlich kleiner. Durch die zweistufige Partialkondensation bei einer ersten Temperatur und einer zweiten tieferen Temperatur wird insgesamt eine höhere Trennschärfe erreicht als durch eine einstufige Partialkondensation. Außerdem ist die benötigte Kühlleistung bei der zweistufigen Partialkondensation in der Regel geringer als bei einer einstufigen Partialkondensation.

Der nicht kondensierte Brüden umfasst Isobuten mit hohem Reinheitsgrad, welches in die Veresterung a) zurückgeführt werden kann, während die vereinigten Kondensate der fraktionierenden Kondensation nur geringe Mengen von Isobuten aufweisen, die in den nachfolgenden Schritten der Aufarbeitung abgetrennt werden.

### Veresterung

Bei der Veresterung a) wird eine aliphatische C₁-C₄-Carbonsäure mit Isobuten in Gegenwart eines sauren Katalysators zu einem Veresterungsgemisch umgesetzt. Bei den aliphatischen C₁-C₄-Carbonsäuren handelt es sich insbesondere um Ameisensäure, Essigsäure, Propionsäure, Buttersäure und Isobuttersäure. In einer bevorzugten Ausführungsform handelt es sich um Acrylsäure oder Methacrylsäure, wobei Acrylsäure besonders bevorzugt ist.

Das Verfahren erfolgt im Allgemeinen in Abwesenheit eines Lösungsmittels und in flüssiger Phase. Als Katalysatoren verwendet man daher solche, die im Reaktionsgemisch zumindest teilweise löslich sind. Geeignete Katalysatoren sind starke anorganische oder organische Säuren. Starke anorganische Säure sind beispielsweise Mineralsäuren wie Schwefelsäure, Phosphorsäure und Polyphosphorsäure, vorzugsweise Schwefelsäure. Starke organische Säuren sind beispielsweise Sulfonsäuren, wie p-Toluol-, Benzol-, Dodecylbenzol- und Methansulfonsäure, vorzugsweise p-Toluolsulfonsäure und Methansulfonsäure. Insbesondere die anorganischen Katalysatoren sind zu Beginn der Umsetzung nur teilweise im Reaktionsgemisch löslich. Im Laufe der Umsetzung wird der Katalysator besser löslich (in erster Linie auf Grund der Bildung eines Teilesters des Katalysators, z. B. des Schwefelsäurehalbesters). Zumindest im letzten Abschnitt liegt er daher im Allgemeinen im Reaktionsgemisch gelöst vor.

Die Konzentration des Katalysators im Veresterungsgemisch beträgt im Allgemeinen etwa 0,1 bis 10 Gew.-%, vorzugsweise 0,5 bis 5 Gew.-%, bezogen auf die Gesamtmenge des Veresterungsgemischs.

Die Umsetzung der aliphatischen C₁-C₄-Carbonsäure mit Isobuten in Gegenwart eines sauren Katalysators erfolgt in herkömmlichen Reaktionsbehältern oder in Säulen (DE-A-11 28 428). Ein geeigneter Reaktor ist in der WO 02/10109 A1 beispielhaft beschrieben.

Bevorzugt wird die Umsetzung in einem Reaktor durchgeführt, bei dem es sich insbesondere um einen zylindrischen Reaktor handelt. Der Reaktor ist in mehrere, vorzugsweise 3, 4 oder 5, voneinander getrennte Abschnitte unterteilt. Die Abschnitte werden durch Trennwände, die senkrecht zur Längsachse des Reaktors verlaufen, voneinander getrennt. Diese weisen jeweils mindestens eine Öffnung auf, um den Durchtritt des Reaktionsgemisches von einem Reaktorabschnitt zum nächsten zu ermöglichen. Die Zahl der Öffnungen pro Trennwand richtet sich nach der Größe des Reaktors. Vorzugsweise weisen die Trennwände eine Öffnung auf, die sich insbesondere in der Mitte der Trennwand befindet. Die Gesamtfläche der Öffnungen pro Trennwand beträgt etwa 1/2000 bis 1/500 der Reaktorquerschnittsfläche.

Das Volumen der Reaktorabschnitte kann gleich oder verschieden sein. Vorzugsweise ist das Volumen des ersten Reaktorabschnitts größer als das der restlichen Abschnitte. Bei einem Reaktor mit vier Abschnitten haben sich folgende Anteile der einzelnen Abschnitte am Gesamtreaktorvolumen als bevorzugt erwiesen:

| | |
|---|---|
| Reaktorabschnitt 1 | 25 bis 50% |
| Reaktorabschnitt 2 | 10 bis 25% |
| Reaktorabschnitt 3 | 10 bis 25% |
| Reaktorabschnitt 4 | 25 bis 50% |

Die Reaktorabschnitte können vorteilhaft mit Einbauten ausgestattet sein, um die Durchmischung des Reaktionsvolumens zu verbessern. Geeignete Einbauten sind beispielsweise statische Mischelemente und ähnlich wirkende Einbauten, wie Gitterroste, Verteilerbleche oder Siebböden. Besonders bevorzugt ist es, den ersten Reaktorabschnitt mit derartigen Einbauten auszurüsten, die dort dann insbesondere in der oberen Hälfte des Reaktorabschnittes vorgesehen werden.

Die C₁-C₄-Carbonsäure wird in flüssiger Form in den ersten Abschnitt des Reaktors eingespeist, insbesondere im Bereich des Bodens des Reaktors. Die Einspeisung kann direkt, z. B. über ein Tauchrohr erfolgen, es ist jedoch bevorzugt, Mittel vorzusehen, welche eine gleichmäßige Verteilung und Durchmischung der Einsatzstoffe ermöglichen. Derartige Mittel sind dem Fachmann bekannt, beispielsweise handelt es sich um Verteilerplatten, perforierte Platten und Rohre, Düsen, etc. Die C₁-C₄-Carbonsäure wird vorzugsweise über eine Düse eingespeist, welche die Vermischung eines Gases und einer Flüssigkeit und die Durchmischung des Reaktorinhalts bewirkt. Sie ist vorzugsweise im Boden des Reaktors angeordnet. Geeignete Düsen sind dem Fachmann bekannt (Strahldüse, Mischdüse, Zweistoffdüse, etc.) und z. B. in Ullmann's Encyclopedia of Industrial Chemistry, Vol. B4, 5. Ausgabe, 1992, S. 280 beschrieben. Insbesondere bei Verwendung einer derartigen Düse ist die Strömung in den beiden ersten Reaktorabschnitten turbulent und in den folgenden Reaktorabschnitten aber im Wesentlichen laminar. Dies erlaubt die Kaskadierung von Reaktionsabschnitten unterschiedlicher Charakteristik, z. B. turbulent mit hoher Rückvermischung wie beim Typ Rührkessel oder laminar mit geringer Rückvermischung wie beim Typ Rohrreaktor, wodurch der jeweilige Reaktionsabschnitt besonders vorteilhaft gestaltet werden kann.

Der Katalysator wird im Gemisch mit der Carbonsäure eingespeist, wobei frischer Katalysator oder zurückgewonnener Katalysator oder ein Gemisch davon zur Anwendung kommen kann.

Es hat sich als vorteilhaft erwiesen, zumindest einen Teil der flüssigen Schwersiederphase aus der partiellen Verdampfung und/oder zumindest einen Teil des Sumpfprodukts der nachstehend beschriebenen Reindestillation in den Reaktor einzuspeisen. Auf diese Weise werden eine Hauptmenge des sauren Katalysators und der nicht umgesetzten Carbonsäure recycliert.

Das Isobuten kann flüssig und/oder gasförmig eingespeist werden. Es wird vorzugsweise über ein ringförmiges Rohr mit mehreren Austrittsöffnungen eingespeist.

Außerdem hat es sich als vorteilhaft erwiesen, bei der Leichtsiederabtrennung anfallendes Rück-Isobuten in den ersten Reaktorabschnitt einzuspeisen. Bei Verwendung von gasförmigem Isobuten ist es besonders vorteilhaft, das Isobuten über die erwähnte Düse zusammen mit der Carbonsäure einzuspeisen. Die Düse bewirkt ein selbsttätiges Ansaugen des zurückgeführten gasförmigen Isobutens, wobei die flüssige Carbonsäure als Treibmedium verwendet wird.

Aus dem ersten und/oder zweiten Reaktorabschnitt kann ein Teil des Reaktionsgemisches entnommen und wieder in den betreffenden Abschnitt zurückgeführt werden. Dadurch wird eine bessere Durchmischung des Reaktionsgemisches gewährleistet. Die Rückführung des Teilstroms erfolgt zweckmäßig über die oben erwähnte Mischdüse in den ersten Reaktorabschnitt und/oder über eine weitere Düse im Bereich der in der Trennwand befindlichen Öffnung in den zweiten Reaktorabschnitt. Bei der weiteren Düse kann es sich um eine Düse des oben für die Mischdüse genannten Typs handeln. Bevorzugt wird eine kegelförmige Düse verwendet. Vorzugsweise ist sie so angeordnet, dass sich ihre Austrittsöffnung etwa in Höhe der Trennwand befindet, die den ersten vom zweiten Abschnitt trennt. Zur Kontrolle bzw. Regelung der Temperatur kann der jeweils entnommene Teilstrom über einen Wärmeübertrager geführt werden.

Das erhaltene Veresterungsgemisch wird am oberen Ende des Reaktors entnommen und der weiteren Aufarbeitung zugeführt. Nicht umgesetztes, gasförmiges Isobuten sammelt sich im oberen Bereich des Reaktors an. Vorzugsweise werden aus dem am oberen Ende des Reaktors abgezogenen Isobuten-haltigen Gasstrom kondensierbare organische Verbindungen, wie nicht umgesetzte Carbonsäure, auskondensiert und so von gegenüber der Veresterung inerten Gasen, wie Luft und Butan, befreit. Nicht umgesetztes Isobuten löst sich teilweise in den auskondensierten Bestandteilen. Die kondensierten organischen Verbindungen werden dann in flüssiger Form beispielsweise über die Mischdüse in den ersten Reaktorabschnitt eingespeist.
Die Veresterungstemperatur liegt insgesamt im Bereich von etwa 10 bis 40 °C. Sie wird vorzugsweise so gesteuert, dass sie im ersten Reaktorabschnitt am höchsten ist. Vorzugsweise liegt die Reaktionstemperatur im ersten Reaktorabschnitt im Bereich von etwa 30 bis 40 °C. Im zweiten Abschnitt ist sie niedriger, vorzugsweise um etwa 5 bis 15 °C. Die Temperatur in den nach dem zweiten Abschnitt folgenden Abschnitten kann gleich oder verschieden sein. Sie ist im Allgemeinen nicht höher als im zweiten Abschnitt, vorzugsweise ist sie niedriger, insbesondere um etwa 3 bis 10 °C. Im vierten Abschnitt ist sie im Allgemeinen so hoch wie im dritten Abschnitt oder um etwa 1 bis 5 °C niedriger. Die Temperatur im letzten Reaktorabschnitt liegt vorzugsweise im Bereich von etwa 10 bis 25 °C.

Die Temperaturverteilung in einem Reaktor mit 4 Abschnitten ist vorzugsweise wie folgt:

| | |
|---|---|
| 1. Abschnitt: | 33 bis 38 °C |
| 2. Abschnitt: | 23 bis 28 °C |
| 3. Abschnitt: | 15 bis 22 °C |
| 4. Abschnitt: | 15 bis 22 °C |

Die Temperatur im 3. und 4. Abschnitt kann dabei gleich oder verschieden sein.

Da die Addition von Carbonsäuren an Isobuten exotherm ist, ist es zur Einstellung der Reaktionstemperatur zweckmäßig, zumindest in den ersten beiden Reaktorabschnitten die Reaktionswärme abzuführen. Dies erfolgt insbesondere mit Hilfe von Wärmetauschern, die außen- oder innenliegend ausgeführt sein können. Auch eine Kühlung der Reaktorwände ist möglich. Es hat sich als zweckmäßig erwiesen, die Temperaturkontrolle in den beiden ersten Reaktorabschnitten mit Hilfe von außenliegenden Wärmetauschern vorzunehmen, über die ein Teilstrom des in dem jeweiligen Reaktorabschnitt befindlichen Reaktionsgemisches geführt und wieder zurückgeführt wird.

Die Veresterung kann mit Unterdruck, drucklos oder unter leichtem Überdruck (100 bis 300 mbar abs.), oder vorzugsweise mit Überdruck (z. B. 0,5 bis 3 bar) durchgeführt werden.

Das aus dem Reaktor austretende Reaktionsgemisch enthält einen hohen Anteil an dem gewünschten Ester. Daneben enthält es nicht umgesetzte Reaktanden, Katalysator, Stabilisator, Ester der Katalysatorsäure und weitere geringfügige Nebenprodukte. Das Reaktionsgemisch enthält nur sehr geringe Mengen an Isobuten-Oligomerisierungsprodukt, im Allgemeinen < 2 Gew.-%, bezogen auf das Reaktionsgemisch.

### Katalysatorabtrennung

Zur Abtrennung des Katalysators wird das Veresterungsgemisch partiell verdampft, wodurch man eine den sauren Katalysator enthaltende flüssige Schwersiederphase und einen tert-Butylester und Isobuten enthaltenden ersten Brüden erhält. Der erste Brüden enthält daneben geringe Mengen an Carbonsäure und leichtsiedende Bestandteile (tert-Butanol und Diisoolefin). Die flüssige Schwersiederphase wird im Allgemeinen zumindest teilweise in den Reaktor zurückgeführt.

Die partielle Verdampfung b) kann auf beliebige Weise durchgeführt werden, wird aber bevorzugt zweistufig durchgeführt. Die Verdampfung erfolgt im Allgemeinen bei erhöhter Temperatur und bei verringertem Druck. Die Bedingungen richten sich nach dem jeweils gewünschten Produkt. Sie werden in der Regel so gewählt, dass die Temperatur im Bereich von etwa 50 bis 150 °C liegt. Der Druck wird so eingestellt, dass die Verdampfung rasch und schonend erfolgt. Der Druck liegt z.B. im Bereich von 10 bis 200 mbar abs., besonders bevorzugt im Bereich von 30 bis 90 mbar abs., ganz besonders bevorzugt im Bereich von 50 bis 70 mbar abs..

Zur Erzeugung des Vakuums sind jegliche Vakuumpumpen geeignet. Zur Vermeidung von Kontaminationen hat es sich bewährt, Schmieröl-freie Pumpen zu verwenden. Besonders bevorzugt werden Wälzkolbenvakuumpumpen ohne Schmieröl und sogenannte trockenlaufende Schrauben-Vakuumpumpen verwendet. Alternativ können Flüssigkeitsringpumpen verwendet werden, in denen z. B. der Zielester als Sperrflüssigkeit dient.

Die zweistufige Verdampfung wird vorzugsweise so durchgeführt, dass in der ersten Stufe 40 bis 95 Gew.-%, vorzugsweise 60 bis 90 Gew.-% des gewünschten Esters abdampfen. Der Brüden enthält neben dem tert-Butylester und Carbonsäure die leichtsiedenden Bestandteile, wie tert-Butanol, tert-Butylacetat und Diisobuten. Der in der ersten Destillation anfallende Sumpf umfasst als erste Schwersiederphase im Wesentlichen den restlichen tert-Butylester, Carbonsäure, sauren Katalysator und hochsiedende Bestandteile, beispielsweise polymere (Meth)acrylverbindungen bei Verwendung von (Meth)acrylsäure. 10 bis 100 Gew.-% der ersten Schwersiederphase werden der zweiten Verdampfungsstufe zugeführt. Falls nur ein Teil der ersten Schwersiederphase der zweiten Verdampfungsstufe zugeführt wird, wird der Rest der ersten Schwersiederphase in den Reaktor zurückgeführt. In der zweiten Verdampfungsstufe werden der restliche Zielester und die Hauptmenge an Carbonsäure (bis zu etwa 90 Gew.-%) abgedampft. Die Brüden beider Stufen werden vereinigt und als erster Brüden weitergeführt.

Der Sumpf der zweiten Verdampfungsstufe umfasst als zweite Schwersiederphase im Wesentlichen den sauren Katalysator, die restliche Carbonsäure und hochsiedenden Bestandteile, beispielsweise polymere (Meth)acrylverbindungen bei der Verwendung von (Meth)acrylsäure. Bei der zweistufigen Verdampfung erfolgt somit eine Auftrennung des Reaktionsgemisches in ein Destillat bzw. ersten Brüden, das im Wesentlichen den Zielester, Carbonsäure und die erwähnten leichtsiedenden Bestandteile umfasst und in einen Rückstand (zweite Schwersiederphase), der im Wesentlichen den sauren Katalysator, Carbonsäure und die erwähnten hochsiedenden Bestandteile umfasst. Das Destillat enthält im Allgemeinen < 20 ppm, insbesondere < 10 ppm, Katalysator.

Die zweite Schwersiederphase wird zumindest teilweise ausgeschleust, vorzugsweise vollständig. Sie kann jedoch auch teilweise in den Reaktor zurückgeführt werden.

Beide Verdampfungsstufen können in üblichen Vorrichtungen durchgeführt werden. Vorzugsweise verwendet man jedoch Vorrichtungen, die eine rasche Destillation erlauben, beispielsweise Filmverdampfer. Geeignete Filmverdampfer sind dem Fachmann bekannt, siehe beispielsweise Ullmann's Encyclopedia of Industrial Chemistry, 5th Ed., Vol. B3,2-21 bis 2-24 sowie 3-1 bis 3-25,1988.

Vorzugsweise verwendet man in der ersten Verdampfungsstufe Fallfilm- oder Fallstromverdampfer und in der zweiten Stufe Dünnschichtverdampfer mit Wischern oder Wendelrohrverdampfer.

Als besonders bevorzugt hat es sich erwiesen, in der ersten Verdampfungsstufe einen wie in der WO 02/10110 beispielhaft beschriebenen Fallfilmverdampfer zu verwenden.

### Fraktionierende zweistufige Kondensation

Erfindungsgemäß erfolgt eine zweistufige Partialkondensation des ersten Brüdens bei zwei unterschiedlichen Temperaturen. Hierdurch wird eine möglichst vollständige Abtrennung des tert-Butylesters bei möglichst geringer Co-Kondensation von nicht umgesetztem Isobuten erreicht. Die fraktionierende Kondensation erfolgt bevorzugt in zwei in Serie geschaltete Kondensatoren (einem ersten und einem zweiten Kondensator), insbesondere Platten- oder Rohrbündelkondensatoren. Vorzugsweise verwendet man Rohrbündelkondensatoren mit senkrecht angeordneten Rohren, die vom Brüden von oben nach unten angeströmt werden.

Die erste Temperatur liegt bei 0 bis 45 °C, vorzugsweise 5 bis 35 °C, unterhalb der Kondensationstemperatur des tert-Butylesters beim ersten Druck. Die zweite Temperatur liegt bei 45 bis 80 °C, vorzugsweise 50 bis 65 °C, ganz besonders bevorzugt 50 bis 55 °C unterhalb der Kondensationstemperatur des tert-Butylesters beim zweiten Druck-

Die zweite Temperatur liegt mindestens 5 °C unterhalb der ersten Temperatur. Vorzugsweise liegt die zweite Temperatur mindestens 10 °C, besonders bevorzugt mindestens 20 °C, ganz besonders bevorzugt mindestens 30 °C und am bevorzugtesten mindestens 40 °C unterhalb der ersten Temperatur.

Die erste Temperatur liegt bevorzugt im Bereich von 15 bis 45 °C, besonders bevorzugt im Bereich von 20 bis 40 °C. Die zweite Temperatur liegt bevorzugt im Bereich von -10 bis -25 °C, besonders bevorzugt im Bereich von -15 bis -22 °C.

Der erste Druck liegt bevorzugt im Bereich von 10 bis 200 mbar abs., besonders bevorzugt im Bereich von 30 bis 90 mbar abs., ganz besonders bevorzugt im Bereich von 50 bis 70 mbar abs. Der zweite Druck liegt bevorzugt im Bereich von 10 bis 200 mbar abs., besonders bevorzugt im Bereich von 30 bis 90 mbar abs., ganz besonders bevorzugt im Bereich von 50 bis 70 mbar abs.. Meist sind der erste und zweite Kondensator gasseitig verbunden. Der erste und zweite Druck sind dann gleich.

Beispielsweise beträgt die Kondensationstemperatur von tert-Butylacrylat bei 60 mbar abs. 43 °C. In diesem Fall kann für die Kühlung des ersten Kondensators zweckmäßigerweise Flusswasser oder gleichwertig temperiertes Kühlwasser verwendet werden, während der zweite Kondensator mit Solekühlung betrieben wird.

Zur Vermeidung von Feststoffablagerungen an den Kontaktflächen des Kondensators wird zweckmäßigerweise ein Teilstrom des gesammelten Kondensats umgewälzt, um die Kontaktflächen fortwährend zu spülen. Das umgepumpte Kondensat kann z.B. mittels eines Verteilers gleichmäßig über die Rohre des Rohrbündelkondensators verteilt werden und läuft an den Innenwänden der Rohre des Kondensators hinab. Beim Einsatz polymerisationsfähiger Carbonsäuren wird auf diese Weise auch eine Verteilung eines nachstehend näher beschriebenen Stabilisators erzielt.

Das Kondensat des ersten und zweiten Kondensators kann getrennt gesammelt und umgewälzt werden. Im Allgemeinen pumpt man jedoch Teilströme des vereinigten Kondensats in beide Kondensatoren. In einer bevorzugten Ausführungsform erfolgt die Zufuhr des Kondensats in den ersten bzw. zweiten Kondensator jeweils über das zuführende Brüdenrohr. Vorzugsweise erfolgt das Einbringen des Kondensats durch Eindüsen in das Brüdenrohr in Gegenrichtung zum Gasstrom, wobei die Düse zweckmäßigerweise im Brüdenrohr im Bereich des Kondensatoreingangs eingebaut ist. Die Menge an Kondensat, die in den ersten Kondensator zurückgeführt wird, beträgt bevorzugt etwa die 5 bis 10-fache Menge des der weiteren Aufarbeitung zugeführten Stroms. Die Menge an Kondensat, die in den zweiten Kondensator zurückgeführt wird, beträgt bevorzugt etwa 0,1 bis 5% des in den ersten Kondensator zurückgeführten Kondensats.

### Leichtsiederabtrennung

Das vereinigte Kondensat der fraktionierenden Kondensation c) wird einer gemeinsamen Aufarbeitung zugeführt. Diese Aufarbeitung umfasst im Allgemeinen eine destillative Leichtsiederabtrennung und eine Reindestillation. Bei der destillativen Leichtsiederabtrennung werden die leichtsiedenden Bestandteile, d. h. von Isobuten verschiedene Bestandteile, deren Siedepunkt niedriger ist als der des Zielesters, vorzugsweise über Kopf abdestilliert. Bei der Leichtsiederabtrennung fallen außerdem weitere Mengen nicht umgesetztes Isobuten an, die vorzugsweise von den leichtsiedenden Bestandteilen abgetrennt werden und in den Schritt a) zurückgeführt werden. Bei der Herstellung von tert-Butylacrylat fallen als Leichtsieder beispielsweise tert-Butylacetat, tert-Butanol und Diisobuten an.

In einer Ausführungsform führt man zur Leichtsiederabtrennung das vereinigte Kondensat einer ersten Destillationskolonne (D1) zu, wobei man ein erstes flüssiges Sumpfprodukt (S1) und einen vierten Brüden (B4) erhält; das erste flüssige Sumpfprodukt (S1) einer weiteren Aufarbeitung zuführt; den vierten Brüden (B4) fraktionierend kondensiert, indem man den vierten Brüden (B4) bei einem dritten Druck und einer dritten Temperatur partiell kondensiert und ein erstes Leichtsiederkondensat (K3) erhält, den nicht kondensierten fünften Brüden (B5) bei einem vierten Druck und einer vierten Temperatur partiell kondensiert und ein zweites Leichtsiederkondensat (K4) erhält, wobei die vierte Temperatur niedriger ist als die dritte Temperatur; man den bei der vierten Temperatur nicht kondensierten sechsten Brüden (B6) in den Schritt a) zurückführt; und das erste und/oder das zweite Leichtsiederkondensat (K3) und (K4) teilweise als Rücklauf zum Kopf der ersten Destillationskolonne (D1) zurückführt.

Die zweistufige Kondensation ist energetisch vorteilhaft, da in der ersten Stufe ein Kühlmittel auf einem höheren Temperaturniveau verwendet werden kann. Der vierte Brüden wird in der ersten Stufe lediglich auf die dritte Temperatur unter den Taupunkt der Leichtsieder gekühlt. Im fünften Brüden verbliebene, bei der dritten Temperatur nicht kondensierte Leichtsieder werden bei der tieferen vierten Temperatur kondensiert. Auf diese Weise wird auch eine verringerte Co-Kondensation von Isobuten erreicht.

Der sechste Brüden kann auch noch bis zu 5 Gew.-%, bezogen auf das Kopfprodukt, an Zielester enthalten. Das erste flüssige Sumpfprodukt umfasst im Wesentlichen Zielester und Carbonsäure.

Isobuten wird in Form des bei der vierten Temperatur nicht kondensierten sechsten Brüdens abgetrennt und der Veresterung a) zugeführt. Die fraktionierende Kondensation erlaubt hohe Reinheitsgrade der gewonnenen Fraktionen und somit ein effizienteres Verfahren mit verminderten Rückströmen.

Die Destillationstemperatur (Sumpftemperatur) in der ersten Destillationskolonne liegt im Allgemeinen im Bereich von 30 bis 110 °C. Der Druck wird entsprechend je nach Produkt gewählt.

Als Destillationskolonne kommen übliche Kolonnen mit Schüttungen oder gerichteten Packungen oder mit Glocken-, Ventil- oder Siebböden in Betracht. Vorzugsweise verwendet man jedoch eine Bodenkolonne mit 30 bis 50 Dual-Flow-Böden. Der Zulauf zur Destillationskolonne erfolgt im Allgemeinen im mittleren Bereich.

Die fraktionierende Kondensation der leichtsiedenden Komponenten erfolgt vorzugsweise in zwei in Serie geschalteten Kondensatoren (einem dritten und einem vierten Kondensator), insbesondere Platten- oder Rohrbündelkondensatoren. Vorzugsweise verwendet man Rohrbündelkondensatoren mit senkrecht angeordneten Rohren, die vom Brüden von oben nach unten angeströmt werden. Die Temperatur des Kühlmittels des vierten Kondensators ist um etwa 30 bis 60 °C niedriger als die des dritten Kondensators, bei dem das Kühlmittel eine Temperatur im Bereich von etwa 10 bis 35 °C aufweist.

Diisobuten ist Hauptbestandteil der abgetrennten Leichtsieder. Diisobuten ist ein Gemisch verschiedener Isoocten-Isomerer. Deren Kondensationspunkt liegt in der Praxis eng beisammen. Als Bezugspunkt kann z. B. der Kondensationspunkt von 2,4,4-Trimethylpent-1-en gelten.

Die dritte Temperatur liegt bevorzugt bei 5 bis 40 °C, besonders bevorzugt 7 bis 30 °C unterhalb der Kondensationstemperatur von Diisobuten beim dritten Druck. Die vierte Temperatur liegt bevorzugt bei 30 bis 55 °C, besonders bevorzugt bei 35 bis 50 °C, ganz besonders bevorzugt 35 bis 45 °C unterhalb der Kondensationstemperatur von Diisobuten beim vierten Druck

Die vierte Temperatur liegt mindestens 5 °C unterhalb der dritten Temperatur. Vorzugsweise liegt die vierte Temperatur mindestens 10 °C, besonders bevorzugt mindestens 20 °C, ganz besonders bevorzugt mindestens 30 °C und am bevorzugtesten mindestens 40 °C unterhalb der dritten Temperatur.

Der dritte Druck liegt bevorzugt im Bereich von 10 bis 300 mbar abs., besonders bevorzugt im Bereich von 90 bis 150 mbar abs., ganz besonders bevorzugt im Bereich von 110 bis 130 mbar abs. Der vierte Druck liegt bevorzugt im Bereich von 10 bis 200 mbar abs., besonders bevorzugt im Bereich von 90 bis 150 mbar abs., ganz besonders bevorzugt im Bereich von 110 bis 130 mbar abs.. Meist sind der dritte und vierte Kondensator gasseitig verbunden. Der dritte und vierte Druck sind dann gleich.

Beispielsweise beträgt die Kondensationstemperatur von Diisobuten bei 120 mbar abs. 40 °C. In diesem Fall kann für die Kühlung des dritten Kondensators zweckmäßigerweise Flusswasser oder gleichwertig temperiertes Kühlwasser verwendet werden, während der vierte Kondensator mit Solekühlung betrieben verwendet wird.

### Reindestillation

Bei der Reindestillation wird der Zielester von verbliebenen höhersiedenden Komponenten abgetrennt und vorzugsweise über Kopf abdestilliert.

Der tert-Butylester siedet in der Regel bei niedrigeren Temperaturen als die C₁-C₄-Carbonsäure. Das zweite flüssige Sumpfprodukt enthält verbliebene, nicht umgesetzte C₁-C₄-Carbonsäure und wird zumindest teilweise, insbesondere vollständig in die Veresterung a) zurückgeführt.

Die Destillationstemperatur liegt im Allgemeinen im Bereich von 40 bis 130 °C. Der Druck wird entsprechend dem zu destillierenden Ester gewählt.

Bei der zweiten Destillationskolonne handelt es sich üblicherweise um eine herkömmliche Bodenkolonne, beispielsweise eine Kolonne mit 30 bis 50 Dual Flow-Böden und Zulauf im mittleren Kolonnenbereich. Der im Wesentlichen reine Zielester wird über den Kopf der Kolonne abgetrennt.

In einer Ausführungsform führt man das bei der Leichtsiederabtrennung erhaltene erste flüssige Sumpfprodukt (S1) einer zweiten Destillationskolonne (D2) zu, wobei man ein zweites flüssiges Sumpfprodukt (S2) und einen siebten Brüden (B7) erhält; das zweite flüssige Sumpfprodukt (S2) zumindest teilweise in den Schritt a) zurückführt; den siebten Brüden (B7) fraktionierend kondensiert, indem man den siebten Brüden (B7) bei einem fünften Druck und einer fünften Temperatur partiell kondensiert und ein erstes Produktkondensat (P1) erhält, den nicht kondensierten achten Brüden (B8) bei einem sechsten Druck und einer sechsten Temperatur partiell kondensiert und ein zweites Produktkondensat (P2) erhält, wobei die sechste Temperatur niedriger ist als die fünfte Temperatur; und das erste und/oder das zweite Produktkondensat (P1) und (P2) teilweise als Rücklauf in die zweite Destillationskolonne (D2) zurückführt.

Die Teilströme des ersten und zweiten Produktkondensats, welche nicht als Rücklauf in die zweite Destillationskolonne zurückgeführt werden, werden zumindest teilweise als Produkt aus dem Verfahren ausgeführt.

Die fraktionierende Kondensation des Zielesters erfolgt vorzugsweise in zwei seriell angeordneten Kondensatoren (einem fünften und einem sechsten Kondensator), insbesondere Platten- oder Rohrbündelkondensatoren. Vorzugsweise verwendet man Rohrbündelkondensatoren mit senkrecht angeordneten Rohren, die vom Brüden von oben nach unten angeströmt werden. Die zweistufige Kondensation ist energetisch vorteilhaft, da in der ersten Stufe ein Kühlmittel auf einem höheren Temperaturniveau verwendet werden kann. Der siebte Brüden wird in der ersten Stufe lediglich auf die fünfte Temperatur unter den Taupunkt der Zielesters gekühlt. Im achten Brüden verbliebener, bei der fünften Temperatur nicht kondensierter Zielester wird bei der tieferen sechsten Temperatur kondensiert.

Zur Vermeidung von Feststoffablagerungen an den Kontaktflächen der Kondensatoren wird zweckmäßigerweise ein Teilstrom des gesammelten Kondensats umgewälzt, um die Kontaktflächen fortwährend zu spülen. Das umgepumpte Kondensat kann z.B. mittels eines Verteilers gleichmäßig über die Rohre des Rohrbündelkondensators verteilt werden und läuft an den Innenwänden der Rohre des Kondensators hinab. Beim Einsatz polymerisationsfähiger Carbonsäuren wird auf diese Weise auch eine Verteilung eines nachstehend näher beschriebenen Stabilisators erzielt.

Das Kondensat des fünften bzw. sechsten Kondensators kann getrennt gesammelt und umgewälzt werden. Im Allgemeinen pumpt man jedoch Teilströme des vereinigten Kondensats in beide Kondensatoren. In einer bevorzugten Ausführungsform erfolgt die Zufuhr des Kondensats in den fünften bzw. sechsten Kondensator jeweils über das zuführende Brüdenrohr. Vorzugsweise erfolgt das Einbringen des Kondensats durch Eindüsen in das Brüdenrohr in Gegenrichtung zum Gasstrom, wobei die Düse zweckmäßigerweise im Brüdenrohr im Bereich des Kondensatoreingangs eingebaut ist. Die Menge an Kondensat, die in den fünften Kondensator zurückgeschleust wird, beträgt bevorzugt etwa die 5 bis 10-fache Menge des ausgeführten Stroms. Die Menge an Kondensat, die in den sechsten Kondensator zurückgeschleust wird, beträgt bevorzugt etwa 0,1 bis 5% des in den ersten Kondensator zurückgeschleusten Kondensats.

Die Temperatur des Kühlmittels des sechsten Kondensators ist um etwa 30 bis 60 °C niedriger als die des fünften Kondensators, bei dem das Kühlmittel eine Temperatur im Bereich von etwa 10 bis 35 °C aufweist.

Die fünfte Temperatur liegt bevorzugt 0 bis 45 °C, vorzugsweise 5 bis 35 °C, unterhalb der Kondensationstemperatur des tert-Butylesters beim fünften Druck und die sechste Temperatur 45 bis 80 °C, vorzugsweise 50 bis 65 °C, ganz besonders bevorzugt 50 bis 55 °C unterhalb der Kondensationstemperatur des tert-Butylesters beim sechsten Druck.

Die sechste Temperatur liegt mindestens 5 °C unterhalb der fünften Temperatur. Vorzugsweise liegt die sechste Temperatur mindestens 10 °C, besonders bevorzugt mindestens 20 °C, ganz besonders bevorzugt mindestens 30 °C und am bevorzugtesten mindestens 40 °C unterhalb der ersten Temperatur.

Der fünfte Druck liegt bevorzugt im Bereich von 10 bis 200 mbar abs., besonders bevorzugt im Bereich von 30 bis 100 mbar abs., ganz besonders bevorzugt im Bereich von 50 bis 90 mbar abs. Der sechste Druck liegt bevorzugt im Bereich von 10 bis 200 mbar abs., besonders bevorzugt im Bereich von 30 bis 100 mbar abs., ganz besonders bevorzugt im Bereich von 50 bis 90 mbar abs. Meist sind der fünfte und sechste Kondensator gasseitig verbunden. Der fünfte und sechste Druck sind dann gleich.

Die Reinheit des erhaltenen Zielesters liegt üblicherweise bei 99,5 bis 99,9 Gew.-% Zielester.

### Anfahren und Instandhaltung des Reaktors

Im stationären Zustand liegen im Reaktor die Edukte als Lösung im Zielester vor, was eine Vergleichmäßigung der Reaktion und eine besonders vorteilhafte Wärmeabfuhr erlaubt. Zum Anfahren des Reaktors wird der Reaktor daher bevorzugt mit dem Zielester befüllt. Im Anschluss werden die Edukte und der Katalysator in den Reaktor eingeleitet und die Reaktion beginnt.

Beim Abfahren der Anlage wird der Reaktorinhalt bevorzugt in einen Auffangbehälter geleitet. Der Auffangbehälter ist am geodätisch tiefsten Punkt der Anlage angeordnet und über separate Leitungen mit dem Reaktor verbunden. Im Falle einer Leckage ist so ein schnelles Entleeren des Reaktors möglich. Üblicherweise sind hierzu keine Pumpsysteme notwendig. Der Auffangbehälter verfügt über einen Druckausgleich und ist mit einem sauerstoffhaltigen Gas mit einem Sauerstoffgehalt von 10 Vol.-% Sauerstoff oder weniger, vorzugsweise 5 Vol.-% Sauerstoff oder weniger, in Inertgas, vorzugsweise Stickstoff, befüllt. Die Kühlung des Auffangbehälters erfolgt mittels einer Pumpe und einem externen Wärmetauscher. Der Inhalt des Auffangbehälters kann dann unabhängig weiter aufgearbeitet werden.

Die Edukte, insbesondere die C₁-C₄-Carbonsäure, werden vorzugsweise in weitgehend wasserfreier Form eingesetzt. Die im Verfahren mit den Reaktionskomponenten in Kontakt stehenden Oberflächen bestehen bevorzugt aus Materialien, die bezüglich technischer Korrosionsbeständigkeit an die Korrosivität der eingesetzten Carbonsäure angepasst sind, wie z. B. Edelstahl der Qualität 1.4541, 1.4571 bzw. diesen im Korrosionsverhalten mindestens gleichwertigen Edelstähle. Aufgrund des sehr niedrigen Wassergehalts im Prozesssystem kommt es bei diesen Werkstoffen auch bei Einsatz von starken anorganischen Säuren als Katalysator zu keinem über das Maß der technischen relevanten Beständigkeit hinausgehenden Korrosionsangriff. In Produktionsanlagen für ethylenisch ungesättigte Ester besteht üblicherweise die Notwendigkeit zur Reinigung mit heißer Natronlauge, wodurch die verwendeten Werkstoffe eine Wechselbelastung zwischen organischer Säure und dem Reinigungsmedium Natronlauge erfahren. Der Einsatz von sogenannten Duplexstählen wie 1.4462 für eine verbesserte Langzeitbeständigkeit der apparativen Ausrüstung kann daher von Vorteil sein.

Insbesondere in den Bereichen, wo neben der beschriebenen korrosiven Belastung durch organische Säuren und einer starken anorganischen Säure als Katalysator zusätzlich noch eine hohe Temperatur und eine mechanische Belastung vorliegen, wie im Dünnschichtverdampfer zur Abtrennung des sauren Katalysators vom Hauptteil der organischen Substanz, ist es von Vorteil, deutlich besser korrosionsbeständige Werkstoffe, z. B. Nickel-Basiswerkstoffe wie 2.4602, 2.4605, 2.4610 oder 2.4819, einzusetzen. Diese Werkstoffe besitzen neben der erfahrungsgemäß besseren Langzeithaltbarkeit aufgrund noch kleinerer korrosiver Abtragsraten gegenüber den Edelstählen darüber hinaus auch erhebliche Reserven im Falle von unplanmäßigem Auftreten von Wasser als korrosionsförderndem Agens. Die Verwendung dieser Werkstoffe erlaubt vorteilhafte Notbetriebseigenschaften ohne zu befürchtenden schnellen Totalverlust von Apparaten. Wasser kann abweichend vom Regelbetrieb z. B. durch temporäre ungewollte Einschleppung in das System anwesend sein, beispielsweise durch wasserbelastete Einsatz- oder Hilfsstoffe, durch eine Leckage in der Reaktorkühlung oder in den zur fraktionierten Kondensation verwendeten Kondensatoren, oder aufgrund einer Dampfleckage in den Prozess an den direkt mit Dampf beheizten Apparaten.

Zur Reinigung des Reaktors wird der entleerte Reaktor bevorzugt mit auf ca. 80 °C erwärmter Natriumhydroxid-Lösung (z. B. 5 Gew.-% in vollentsalztem Wasser) befüllt und die Lösung im Reaktor umgewälzt. Die nach der Reinigung verbleibende, abgekühlte Lauge wird verworfen, gegebenenfalls nach einer geeigneten Behandlung zur Abgabe in eine Abwasserbehandlungseinrichtung (z. B. einer Kläranlage). Nach der Reinigung des Reaktors, insbesondere von organischen Verschmutzungen, können Reste der Lösung im Reaktorsystem bzw. weiteren gereinigten Anlagenteilen mittels Spülung mit Wasser entfernt werden.

### Sicherheitseinrichtungen

Isobuten ist hochentzündlich und kann im Beisein von Sauerstoff explosive Gemische bilden, die sich in Gegenwart bestimmter Sauerstoffkonzentrationen an heißen Oberflächen entzünden können. Die Anlage wird im Regelbetrieb sowie bei An- und Abfahrvorgängen in geeigneter Weise so betrieben, dass die Sauerstoff-Konzentration in der Gasphase zu jedem Zeitpunkt unterhalb der für eine Explosion erforderlichen Sauerstoffkonzentration liegt. Hierzu wird die Anlage vor der Inbetriebnahme bevorzugt mit einem sauerstoffhaltigen Gas mit einem Sauerstoffgehalt von 10 Vol.-% Sauerstoff oder weniger, vorzugsweise 6 Vol.-% Sauerstoff oder weniger, im Gemisch mit einem Inertgas, vorzugsweise Stickstoff, durchspült und befüllt. Vorzugsweise handelt es sich bei dem sauerstoffhaltigen Gas um sogenannte Magerluft mit einem Sauerstoffgehalt von 10 Vol.-% Sauerstoff oder weniger, hergestellt z. B. durch geeignete Verdünnung von Luft mit z. B. molekularem Stickstoff. Alle dem Prozess zuzuführenden Komponenten werden bevorzugt unter einer Magerluft-Atmosphäre zugeführt. Ein völliger Sauerstoffausschluss ist insbesondere dann nicht erwünscht, wenn einer der nachstehend erläuterten Stabilisatoren Sauerstoff zur Entfaltung seiner Wirksamkeit benötigt. Wird Sauerstoff während des Verfahrens verbraucht, so wird an geeigneten Stellen bevorzugt kontinuierlich frische Magerluft eingespeist, z. B. in den Sumpf der zweiten Destillationskolonne. Die Verwendung von Magerluft vermeidet, dass auch bei Inhomogenitäten in der Zusammensetzung der Gasphase ein explosionsgefährlicher Bereich der Gaszusammensetzung durchschritten wird.

Um Leckagen von Luft, insbesondere in unter Unterdruck betriebene Anlagenkomponenten hinein, zu detektieren, sind bevorzugt an verschiedenen Stellen der Anlage online Sauerstoffmessungen installiert. Besonders bevorzugt werden diese online Sauerstoffmessungen in den Leitungen der nicht kondensierbaren Brüden der fraktionierenden Kondensationen installiert.

Der Reaktor ist vollständig mit Flüssigkeit befüllt und wird daher bevorzugt mit einem Sicherheitsventil gegen thermische Ausdehnung abgesichert. Außerdem verfügt der Reaktor bevorzugt über ein Schnellabschott-, Entleer- und Entspannungssystem (SAEES), durch das im Falle einer Leckage der gesamte Reaktorinhalt ohne Kontakt zur Umwelt in einen belüfteten Auffangbehälter abgelassen werden kann, welcher explosionstechnisch sicher beatmet bzw. entlüftet ist. Der Inhalt dieses Auffangbehälters kann bevorzugt über einen Wärmetauscher gekühlt werden, um ggf. durch Nachreaktion entstandene Wärme kontrolliert abführen zu können. Der Auffangbehälter und seine zugeordneten Einrichtungen sind derart gestaltet, dass sein Inhalt bevorzugter Weise dem Prozess an verschiedenen Stellen wieder zugeführt werden kann.

### Stabilisatorzugabe

Die im vorliegenden Verfahren verwendeten C₁-C₄-Carbonsäuren können, wenn es sich um Carbonsäuren mit ethylenisch ungesättigten Gruppen handelt, vor allem bei höherer Temperatur eine starke Polymerisationsneigung aufweisen. Insbesondere bei Destillationen sind diese Verbindungen im Allgemeinen Temperaturen ausgesetzt, die leicht eine unerwünschte radikalische Polymerisation auslösen können. Dies hat zum einen die Verschmutzung der Apparaturen, das Verstopfen von Leitungen und Pumpen und die Belegung von Kolonnenböden und Wärmeaustauscherflächen zur Folge. Das Reinigen der Anlagen ist ein aufwendiger, teurer und umweltbelastender Vorgang, und die Verfügbarkeit der Anlagen wird dadurch stark reduziert. Zum anderen können unkontrollierte radikalische Polymerisationen ein Sicherheitsrisiko darstellen. Der Einsatz geeigneter Stabilisatoren kann derartige Polymerisationen unterbinden.

Zur Polymerisationsinhibition werden üblicherweise Stabilisatoren bzw. Inhibitoren verwendet. Diese Stabilisatoren sind typischerweise Feststoffe und werden dem Verfahren in Lösung zugeführt. Das Herstellen der Stabilisatorlösungen erfolgt bevorzugt diskontinuierlich als Batch-Ansatz.

Geeignete Stabilisatoren sind beispielsweise N-Oxyl-Verbindungen, Nitrosoverbindungen, Phenolverbindungen, Phenothiazine oder Gemische davon. Die polymerisationsinhibierende Wirkung der Stabilisatoren wird in der Regel durch die Gegenwart von molekularem Sauerstoff verstärkt. In einigen Fällen ist die Anwesenheit von molekularem Sauerstoff für die effektive Wirksamkeit des Stabilisators zwingend erforderlich. Es ist daher bevorzugt, dass in der Anlage molekularer Sauerstoff vorhanden ist.

Geeignete N-Oxyl-Verbindungen umfassen 1-Oxyl-2,2,6,6-tetramethylpiperidin (TEMPO), 1-Oxyl-2,2,6,6-tetramethylpiperidin-4-ol (4-HT), 1-oxyl-2,2,6,6-tetramethylpiperidin-4-on, 1-Oxyl-2,2,6,6-tetramethyl-4-n-propoxypiperidin, 1-Oxyl-2,2,6,6-tetramethyl-4-(2-methoxyethoxy)piperidin, 1-Oxyl-2,2,6,6-tetramethyl-4-(2-methoxyethoxyacetoxy)piperidin; 1-Oxyl-2,2,6,6-tetramethylpiperidin-4-yl-stearat, 1-Oxyl-2,2,6,6-tetramethylpiperidin-4-yl-acetat, 1-Oxyl-2,2,6,6-tetramethylpiperidin-4-yl-butyrat, 1-Oxyl-2,2,6,6-tetramethylpiperidin-4-yl 2-ethylhexanoat, 1-Oxyl-2,2,6,6-tetramethylpiperidin-4-yl-octanoat, 1-Oxyl-2,2,6,6-tetramethylpiperidin-4-yl-laurat, 1-Oxyl-2,2,6,6-tetramethyl-piperidin-4-yl-benzoat, 1-Oxyl-2,2,6,6-tetramethylpiperidin-4-yl-4-tert-butylbenzoat, Bis(1-oxyl-2,2,6,6-tetramethylpiperidin-4-yl)-succinat, Bis(1-oxyl-2,2,6,6-tetramethylpiperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-n-butylmalonat, Bis(1-oxyl-2,2,6,6-tetramethylpiperidin-4-yl)phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethyl-piperidin-4-yl)-hexahydroterephthalat, 1-Oxyl-2,2,6,6-tetramethyl-4-allyloxy-piperidin, 1-Oxyl-2,2,6,6-tetramethyl-4-acetamidopiperidin, 1-Oxyl-2,2,6,6-tetramethyl-4-(N-butyl-formamido)piperidin, N,N'-Bis(1-oxyl-2,2,6,6-tetramethylpiperidin-4-yl)adipamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-(N-butyl-N-(1-oxyl-2,2,6,6-tetramethylpiperidin-4-yl]-s-triazin, 4,4'-Ethylenbis(1-oxyl-2,2,6,6-tetramethylpiperazin-3-one), 1-Oxyl-2,2,6,6-tetramethyl-4-(2,3-dihydroxypropoxy)piperidin, 1-Oxyl-2,2,6,6-tetramethyl-4-(2-hydroxy-4-oxapentoxy)piperidin, Di-tert-butylnitroxyl und 4,4',4"-Tris-(2,2,6,6-tetramethylpiperidinooxyl)phosphit.

1-Oxyl-2,2,6,6-tetramethylpiperidin-4-ol (4-HT) ist besonders geeignet.

Geeignete Nitrosoverbindungen umfassen Nitrosophenol, N-Nitrosodiphenylamin, Isoamylnitrit, N-Nitrosocyclohexylhydroxylamin, N-Nitroso-N-phenylhydroxylamin und deren Salze.

Geeignete Phenolverbindungen umfassen Hydrochinon, 2-Methoxyphenol, 3-Methoxyphenol, 4-Methoxyphenol (MEHQ), 2-Ethoxyphenol, 3-Ethoxyphenol und 4-Ethoxyphenol. 4-Methoxyphenol (MEHQ) ist besonders geeignet.

Geeignete Phenothiazine umfassen Phenothiazin (PTZ), 2-Methylphenothiazin, 2-Octylphenothiazin, 2-Nonylphenothiazin, 2,8-Dimethylphenothiazin, 3,7-Dimethylphenothiazin, 3,7-Diethylphenothiazin, 3,7-Dibutylphenothiazin, 3,7-Dioctylphenothiazin und 2,8-Dioctylphenothiazin, 3,7-Dinonylphenothiazin, 2,8-Dinonylphenothiazin, 2-(α,α-Dimethylbenzyl)phenothiazin, 3,7-Bis(α,α-dimethylbenzyl)phenothiazin und 2,8-Bis(α,α-dimethylbenzyl)phenothiazin. Phenothiazin (PTZ) ist besonders geeignet.

Es können auch mehrere Stabilisatoren gleichzeitig zum Einsatz kommen. Die Stabilisatoren kommen im Allgemeinen in Mengen von etwa 2 bis 2000 ppm, bezogen auf die Gesamtmenge an Carbonsäure und Isobuten, zur Anwendung.

Bevorzugter Weise wird der Stabilisator in einem Lösungsmittel gelöst zugegeben. Grundsätzlich geeignet sind alle Lösungsmittel, in denen der jeweilige Stabilisator löslich ist und welches mit der zu stabilisierenden Flüssigphase mischbar ist. Um eine Kontamination mit im Prozess zunächst nicht erforderlichen (externen) Lösungsmitteln bzw. die Erfordernis des Abtrennens eines externen Lösungsmittels zu vermeiden, verwendet man als Lösungsmittel bevorzugt eine im Prozess ohnehin vorhandene Flüssigkeit. Besonders bevorzugt wird der reine Zielester als Lösungsmittel verwendet.

Das Einbringen des Stabilisators erfolgt jeweils in üblicher Weise durch mengenkontrollierte Zufuhr mittels Pumpen, vorzugsweise wird die Stabilisatorlösung zur besseren Verteilung mittels Sprühvorrichtungen wie Spraydüsen eingesprüht.

Einige der genannten Stabilisatoren sind nur in Anwesenheit von Sauerstoff wirksam, beispielsweise MEHQ, wodurch eine relativ hohe Sauerstoffkonzentration, wie sie beispielsweise in Luft vorliegt, vorteilhaft wäre. Andererseits soll die Sauerstoffkonzentration auf verhältnismäßig niedrige Werte begrenzt sein, damit keine explosionsfähigen Gemische auftreten. Das Verfahren wird geeigneter Weise so durchgeführt, dass die Sauerstoff-Konzentration in der Gasphase an allen relevanten Stellen und zu jedem Zeitpunkt unterhalb der Explosionsgrenze liegt. Bevorzugt liegt die Sauerstoffkonzentration in allen gasförmigen Gemischen im Bereich von 3 bis 8 Vol.-%.

Die Polymerisationsneigung besteht vor allem in der flüssigen Phase bei verminderten Konzentrationen an Stabilisatoren und ggf. Sauerstoff. Da die Stabilisatoren in der Regel schwerflüchtig sind, sammeln sie sich bei Verdampfungsschritten im Sumpf der jeweiligen Verdampfungsanlage. Es ist daher normalerweise nötig, nach der Verdampfung von polymerisationsfähigen Verbindungen erneut Stabilisator zuzugeben, wenn die Verbindungen kondensiert werden, da das Kondensat in der Regel weitestgehend frei von Stabilisatoren anfällt.

Das erfindungsgemäße Verfahren umfasst eine Vielzahl an Verfahrensschritten, bei denen Stoffgemische mit sehr unterschiedlichen Zusammensetzungen bei verschiedensten Prozessbedingungen vorliegen. Zur Sicherstellung eines sicheren und wirtschaftlich vorteilhaften Betriebs ist eine Variation der jeweils zugesetzten Stabilisatoren notwendig, welche an verschiedenen Stellen ins Verfahren eingeführt werden.

In einer bevorzugten Ausführungsform ist bei der Umsetzung der C₁-C₄-Carbonsäure mit Isobuten ein unter Phenothiazinen ausgewählter Stabilisator, besonders bevorzugt PTZ, zugegen. Die eingesetzte (Meth)acrylsäure kann bereits mit PTZ vorstabilisiert werden, was insbesondere bei der Inbetriebnahme der Anlage von Vorteil ist. Weitere Mengen PTZ können in den Reaktor dosiert werden. PTZ geht zusammen mit dem sauren Katalysator bei der partiellen Verdampfung b) des Veresterungsgemisches in die flüssige Schwersiederphase über, welche vom produkthaltigen Hauptstrom abgetrennt wird. Die flüssige Schwersiederphase wird vorzugsweise wieder in den Reaktor zurückgeführt, sodass im Allgemeinen nur geringe Ergänzungsmengen an PTZ dem Prozess kontinuierlich frisch zugeführt werden müssen.

In einer bevorzugten Ausführungsform wird ein unter N-Oxyl-Verbindungen ausgewählter Stabilisator, bei der fraktionierenden Kondensation c) zugesetzt. Besonders bevorzugt wird eine Lösung von 4-HT in Zielester zugegeben. Die Zugabe des Stabilisators erfolgt vorzugsweise am Brüdeneintritt in den ersten Kondensator. Mit einem Rückführstrom des Kondensats zum Brüdeneintritt des zweiten Kondensators gelangt dieser Stabilisator auch in den zweiten Kondensator.

Die erste Destillationskolonne wird ebenfalls bevorzugt mit einem unter N-Oxyl-Verbindungen ausgewählten Stabilisator, besonders bevorzugt 4-HT, stabilisiert. Der Zulaufstrom in die erste Destillationskolonne enthält 4-HT aus dem vorhergehenden Schritt, eine weitere Menge 4-HT wird am Kopf der Kolonne zugegeben, insbesondere am Brüdeneintritt des dritten Kondensators und gelangt mit dem Kondensatrücklauf auch in die erste Destillationskolonne.

In einer bevorzugten Ausführungsform wird ein unter N-Oxyl-Verbindungen ausgewählter Stabilisator, besonders bevorzugt 4-HT, dem Zulauf zur zweiten Destillationskolonne zugesetzt.

Der Sumpf und Abtriebsteil der zweiten Destillationskolonne werden durch die N-Oxyl-Verbindung stabilisiert. Es ist bevorzugt, den Verstärkungsteil der zweiten Destillationskolonne nicht mit N-Oxyl-Verbindungen zu stabilisieren, da der Übertritt derartiger Verbindungen nicht vollständig vermeidbar wäre. Die N-Oxyl-Verbindungen sind im Zielester nicht erwünscht, weil sie zu Verfärbungen des Produkts und daraus hergestellten Substanzen führen können. Daher wird im Verstärkungsteil der zweiten Destillationskolonne ein unter Phenolverbindungen ausgewählter Stabilisator, insbesondere MEHQ, zugesetzt. Dieser Stabilisator wird auch zur Stabilisierung des Produkts verwendet und wirkt daher nicht nachteilig bzw. muss nicht in einem späteren Schritt abgetrennt werden. MEHQ wird bevorzugt dem Umwälzstrom über die Kondensatoren und/oder dem Kondensatrücklauf in die zweite Destillationskolonne zugesetzt. Zweckmäßigerweise erfolgt dies durch Eindüsen über eine zentrisch im Brüdenrohrausgang eingebaute Düse.

Um die Wirksamkeit des MEHQ zu gewährleisten, wird in den Sumpf der zweiten Destillationskolonne bevorzugt ein molekularen Sauerstoff enthaltendes Gas, bevorzugt Magerluft (5 Vol.-% Sauerstoff in Stickstoff) eingespeist. Durch diese Maßnahmen ist es möglich, die Polymerisatbildung in den Kondensatoren, den Brüdenrohren und in der Kolonne zu verhindern oder zumindest so weit zu unterdrücken, dass wirtschaftlich vorteilhaft lange Laufzeiten des Betriebs ohne Reinigungsabstellung möglich sind.

Die Erfindung wird durch die beigefügte Figur näher veranschaulicht.

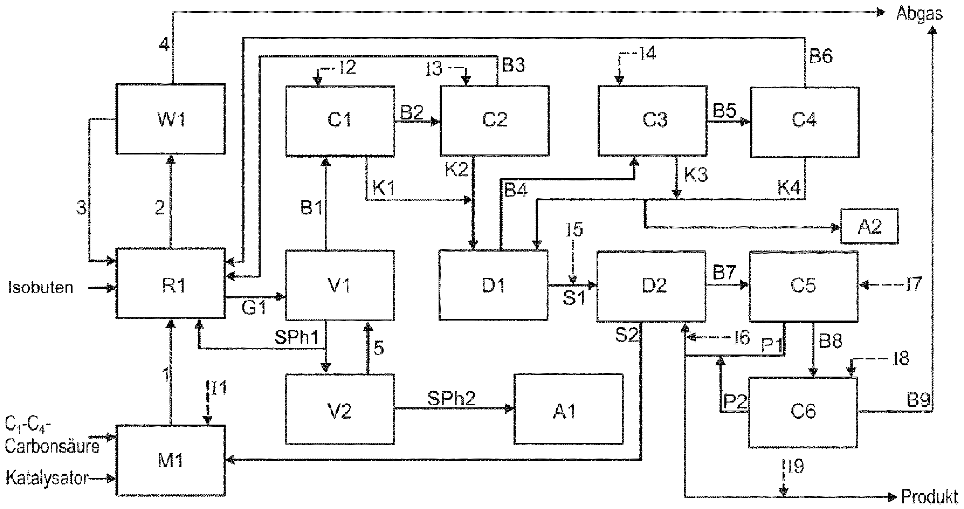

Fig. 1 ist eine schematische Darstellung einer zur Durchführung des erfindungsgemäßen Verfahrens geeigneten Anlage.

Gemäß Fig. 1 werden über einen Mischer M1 eine aliphatische C₁-C₄-Carbonsäure, ein Stabilisator I1 und der saure Katalysator als Gemisch dem Reaktor R1 über eine Leitung 1 und eine Düse E1 (in Fig. 1 nicht dargestellt) zugeführt. Isobuten wird in den Sumpf des Reaktors R1 eingeführt. Über die Düse E1 werden dem Reaktor R1 außerdem die Isobuten-haltigen nicht kondensierten Brüden B3 und B6 der Kondensatoren C2 und C4 zugeführt. Das Kondensat des Rücklaufkühlers W1 wird dem Reaktor R1 zugeführt.

Im Reaktor R1 findet die Additionsreaktion von Isobuten und der aliphatischen C₁-C₄-Carbonsäure statt. Der Reaktor hat vier gekühlte Reaktionszonen. Die Reaktionszonen sind durch Trennbleche voneinander getrennt, wobei der Übergang von einer Reaktionszone zur nächsten aus einem Loch mit geringem Querschnitt besteht. Die Durchmischung der Reaktanden im Reaktor erfolgt durch die Düse E1 und durch Verwirbelung beim Übergang von einer Zone zur nächsten.

Der Abzug des flüssigen Reaktionsprodukts G1 am Kopf des Reaktors R1 erfolgt mittels einer Standregelung, so dass sich eine konstante Flüssig/Gas-Phasengrenze eingestellt. Die im Wesentlichen aus Inertgasen, Isobuten und geringen Mengen des tert-Butylester bestehende Gasphase wird über die Leitung 2 dem Rücklaufkühler W1 zugeführt. Das Kondensat des Rücklaufkühlers W1 enthält Isobuten und Acrylsäure und wird über die Leitung 3 dem Reaktor R1 zugeführt. Die Gasphase des Rücklaufkühlers W1 wird über die Leitung 4 als Abgas aus dem Verfahren ausgeführt.

Das flüssige Reaktionsprodukt G1 wird seitlich am Kopf des Reaktors R1 abgezogen und mengengeregelt der Verdampfungseinheit V1, bestehend aus einem Fallfilmverdampfer und einem Abscheidebehälter (in Fig.1 nicht einzeln dargestellt), zugeführt. Der Druck des flüssigen Reaktionsprodukts wird mittels eines Drosselventils (in Fig. 1 nicht dargestellt) von Reaktordruck auf Unterdruck abgesenkt, bei welchem die folgende Katalysatorabtrennung erfolgt. Im Fallfilmverdampfer der Verdampfungseinheit V1 wird das Reaktionsgemisch partiell verdampft und in den Abscheidebehälter weitergeführt. Der Abscheidebehälter enthält bevorzugt einen Tropfenabscheider, um mitgerissene Schwersiederkomponenten wie Schwefelsäure und den Stabilisator I1 sicher abzutrennen. Die nicht gasförmigen Bestandteile werden im Abscheidebehälter als erste Schwersiederphase SPh1 gesammelt und über einen externen Kühler (in Fig. 1 nicht abgebildet) gekühlt, um eine Rückreaktion des darin enthaltenen tert-Butylesters zur Carbonsäure und Isobuten zu verhindern.

Ein Teil der ersten Schwersiederphase SPh1 wird mengengeregelt dem Dünnschichtverdampfer V2 zugeführt, um die weitere Abtrennung von Carbonsäure bzw. tert-Butylester als Gas zu ermöglichen. Die im Dünnschichtverdampfer V2 erzeugte Gasphase wird über die Leitung 5 in den Abscheidebehälter der Verdampfungseinheit V1 zurückgeführt, während ein Teil der flüssigen zweiten Schwersiederphase SPh2 in den Absetzbehälter A1 geführt wird. Bevorzugt werden Teilströme der zweiten Schwersiederphase SPh2 dazu verwendet, um den Zufuhrstrom zum Dünnschichtverdampfer der Verdampfungseinheit V1 vorzuwärmen. Durch Variation der heißen Teilströme lässt sich die Zusammensetzung des Zufuhrstroms zum Dünnschichtverdampfer der Verdampfungseinheit V1 sowie die Temperatur des Zufuhrstroms variieren.

Ein weiterer Teil der ersten Schwersiederphase SPh1 und ein weiterer Teil der zweiten Schwersiederphase SPh2 werden miteinander oder jeweils einzeln mengengeregelt über die Düse E1 in den Reaktor R1 zurückgeführt (die Rückführung der zweiten Schwersiederphase SPh2 ist in Fig. 1 nicht abgebildet).

Die gasförmigen Bestandteile aus dem Abscheidebehälter der Verdampfungseinheit V1 werden in den Kondensatoren C1 und C2 fraktionierend kondensiert, wobei der Brüden B2 des Kondensators C1 in den Kondensator C2 geführt wird. Ein Stabilisator I2 wird am Kopf des Kondensators C1 zugegeben und ein Stabilisator I3 am Kopf des Kondensators C2 zugegeben. Für die Kühlung des Kondensators C1 kann z. B. Flusswasser oder gleichwertig temperiertes Kühlwasser verwendet werden, während der Kondensator C2 mit Solekühlung betrieben wird. Der im Kondensator C2 nicht kondensierte Brüden B3 wird über die Düse E1 in den Reaktor R1 geführt.

Die in den Kondensatoren C1 und C2 anfallenden Kondensate K1 und K2 werden vereinigt und der Destillationskolonne D1 seitlich zugeführt. In der Destillationskolonne D1 werden Leichtsieder, vor allem Diisobuten und Isobuten, abgetrennt. Die Sumpfbeheizung der Destillationskolonne D1 erfolgt über einen Umlaufverdampfer (in Fig. 1 nicht dargestellt), über den ein Teil des Sumpfs umgepumpt wird. Die Leichtsieder B4 werden dampfförmig am Kopf der Destillationskolonne D1 abgeführt und in den Kondensatoren C3 und C4 fraktionierend kondensiert. Der Brüden B5 des Kondensators C3 wird in den Kondensator C4 geführt. Für die Kühlung des Kondensators C3 kann z. B. Flusswasser oder gleichwertig temperiertes Kühlwasser verwendet werden, während der Kondensator C4 mit Solekühlung betrieben wird. Der im Kondensator C4 nicht kondensierte Brüden B6 wird über die Düse E1 in den Reaktor R1 geführt. Ein Stabilisator I4 wird am Kopf des Kondensators C3 zugegeben. Die in den Kondensatoren C3 und C4 anfallenden Kondensate K3 und K4 werden vereinigt; ein Teilstrom wird als Rücklauf in die Destillationskolonne D1 geführt, der Rest wird dem Absetzbehälter A2 zugeführt.

Der Sumpfstrom S1 der Destillationskolonne D1 wird der Destillationskolonne D2 seitlich zugeführt. In den Zulauf zur Destillationskolonne D2 wird Stabilisator I5 dosiert. Die Sumpfbeheizung der Destillationskolonne D2 erfolgt über einen Umlaufverdampfer (in Fig. 1 nicht dargestellt), über den ein Teil des Sumpfs umgepumpt wird. Im Rahmen des Umpumpens wird dem Sumpf der Destillationskolonne D2 außerdem Magerluft zugeführt.

In der Destillationskolonne D2 wird der tert-Butylester von der verbleibenden aliphatischen Carbonsäure getrennt. Üblicherweise liegt der Siedepunkt der Carbonsäure über dem Siedepunkt des tert-Butylesters, weshalb der reine tert-Butylester über Kopf abgezogen wird und die Carbonsäure am Sumpf der Destillationskolonne D2 anfällt. Um eine Kondensation des tert-Butylesters am Kolonnenkopf zu vermeiden, wird der Kolonnenkopf mit Dampf beheizt. Somit wird auch einer aus der Kondensation ggf. resultierenden Polymerisation des tert-Butylesters vorgebeugt. Der Sumpfstrom S2 der Destillationskolonne D2 wird über einen Wärmetauscher (in Fig. 1 nicht dargestellt) in den Reaktor R1 zurückgeführt.

Der Brüden B7 der Destillationskolonne D2 wird in den Kondensatoren C5 und C6 fraktionierend kondensiert; der Brüden B8 des Kondensators C5 wird in den Kondensator C6 geführt. Ein Stabilisator I7 wird am Kopf des Kondensators C5 zugegeben und ein Stabilisator I8 am Kopf des Kondensators C6 zugegeben. Der im Kondensator C6 nicht kondensierte Brüden B9 wird als Abgas aus dem Verfahren ausgeführt. Das Abgas wird zum Beispiel einer Fackel oder einer Abgasverbrennung zugeführt.

Ein Teilstrom der vereinigten Kondensate P1 und P2 aus den Kondensatoren C5 und C6 wird auf die Kondensatoren C5 und C6 (in Fig. 1 nicht abgebildet) bzw. unter Zusatz des Stabilisators I6 als Rücklauf auf die Destillationskolonne D2 gegeben. Ein weiterer Teilstrom der vereinigten Kondensate P1 und P2 aus den Kondensatoren C5 und C6 wird als reiner tert-Butylester über einen Wärmetauscher (in Fig. 1 nicht abgebildet) aus dem Verfahren ausgeführt. Zur Lagerstabilisierung kann dem reinen tert-Butylester weiterer Stabilisator I9 zugegeben werden.

Die Anlage verfügt bevorzugt über ein Schnellabschott-, Entleer- und Entspannungssystem (SAEES), durch das im Falle eines Lecks der gesamte Inhalt des Reaktors R1 in einen belüfteten Auffangbehälter (in Fig. 1 nicht dargestellt) abgelassen werden kann. Der Inhalt dieses Auffangbehälters kann über einen Wärmetauscher gekühlt werden, um die durch Nachreaktion entstandene Wärme abführen zu können. Der Inhalt des Auffangbehälters kann dem Verfahren an verschiedenen Stellen wieder zugeführt werden, insbesondere dem Reaktor R1, dem Fallfilmverdampfer V2 oder dem Dünnschichtverdampfer der Verdampfungseinheit V1.

### Beispiel 1

Das folgende Beispiel wurde in einer Anlage gemäß Fig. 1 durchgeführt. Alle angegebenen Prozentsätze sind gewichtsbezogen, soweit nicht anders angegeben. Es wurden Acrylsäure und Isobuten unter Zusatz von Schwefelsäure zu tert-Butylacrylat umgesetzt. Die Anlage wurde mit Magerluft (5 Vol.-% Sauerstoff in Stickstoff) durchspült und befüllt.

Acrylsäure (AS, 99,95% mit 0,05% Essigsäure, 0,79 m³/h) wurde in einem Mischer M1 mit der aus dem Sumpf der Destillationskolonne D2 rückgeführten Acrylsäure (93,12% AS, 4,60% Schwersieder, 0,52 m³/h) und mit einer Phenotiazin (PTZ)-Lösung (97,95% AS, 2,00% PTZ, 0,01 m³/h) als Stabilisator I1 vermischt. Schwefelsäure (technisch, 96%, 3,3 kg/h) wurde mittels Stickstoff (3,5 bar abs.) eindosiert. Außerdem wurde ein Teilstrom (2.235 kg/h) der flüssigen Phase aus dem Abscheidebehälter der Verdampfungseinheit V1 dem Gemisch zugeführt.

Die flüssigen Edukte und Rückströme wurden als Gemisch über die Leitung 1, einen Kühler (Rohrbündel, 180 m², Edelstahl 1.4571, in Fig.1 nicht dargestellt) und die Düse E1 dem Reaktor R1, einer kaskadierten Blasensäule, zugeführt. Die Austrittstemperatur des Kühlers betrug 29 °C.

Isobuten (654 kg/h) wurde direkt in den Sumpf des Reaktors dosiert. Außerdem wurden die Isobuten-haltigen Brüden B3 und B6 aus den Kondensatoren C2 und C4 (105 m³/h), zusammen mit dem tert-Butylacrylat-haltigen Kondensat aus dem Rücklaufkühler W1, über die Düse E1 in den Reaktor R1 dosiert. Bei der Düse E1 handelte es sich um eine Ejektorstrahldüse. In der Düse erfolgt eine Druckerhöhung durch den Treibstrahl auf ca. 2 bar abs.

Der Reaktor R1 hatte vier Reaktionszonen, wobei der Übergang aus einem Loch mit geringem Querschnitt (Durchmesser 24 mm) bestand. Die Reaktionszonen wurden jeweils gekühlt (Zone 1: externer Flusswasserkühler mit 121 kW, Zone 2: externer Solekühler mit 28 kW, Zone 3: innenliegender Solekühler mit 14 kW, Zone 4: innenliegender Solekühler mit 14 kW, wobei die Temperatur der Sole je -20 °C betrug). Im Reaktor R1 fand die leicht exotherme Additionsreaktion (-37,6 kJ/mol) von Isobuten und Acrylsäure bei einer Temperatur von 31 °C in der Zone 1, 25 °C in der Zone 2, 20 °C in der Zone 3 und 18 °C in der Zone 4 und einem Druck von 1,93 bar abs. statt.

Die Durchmischung der Reaktanden im Reaktor erfolgte zum einen durch die Düse E1 und zum anderen durch Verwirbelung beim Übergang von einer Zone zur Nächsten. Am Kopf des Reaktors (Zone 4) wurde mittels Standregelung eine Flüssig/Gas-Phasengrenze eingestellt.

Die Gasphase umfasste 25,20% Isobuten, 0,23% tert-Butylacrylat (TBA) und Inertgase und wurde über die Leitung 2 in den Rücklaufkühler W1 geführt. Das im Abgas mitgerissene TBA wurde mittels Rücklaufkühler W1 auskondensiert und über die Leitung 3 im Gemisch mit den Isobuten-haltigen Brüden aus den Kondensatoren C2 und C4 über die Düse E1 in den Reaktor R1 zurückgeführt. Der Druck am Kopf des Reaktors R1 wurde in der Abgasleitung auf 1,2 bar abs. eingestellt. Am Boden des Reaktors stellte sich ein Druck von 1,93 bar abs. ein. Die gasförmigen Bestandteile des Rücklaufkühlers W1 (3,77 m³/h) wurden über die Leitung 4 aus dem Verfahren ausgeführt.

Das flüssige Reaktionsprodukt aus dem Reaktor R1 hatte folgende Zusammensetzung:

| | |
|---|---|
| 4,90% | Isobuten |
| 33,51% | AS |
| 54,00% | TBA |
| 1,00% | Diisobuten |
| 3,71% | Schwersieder |
| 1,56% | Schwefelsäure |
| 1,32% | sonstige Bestandteile |

Das flüssige Reaktionsprodukt aus dem Reaktor R1 (4,83 m³/h, 18 °C) wurde am oberen Ende des Reaktors R1 abgeführt und über einen Siebkorbfilter (0,1 m², in Fig. 1 nicht abgebildet) dem Fallfilmverdampfer (70 °C, 309 kW, 47 m²) der Verdampfungseinheit V1 zugeführt. Über ein Regelventil (Durchflussregelung) wurde der Druck auf 60 mbar abs. reduziert. Es bildete sich ein zweiphasiges Gemisch durch das Verdampfen eines Teils der Leichtsiederkomponenten. Im Fallfilmverdampfer der Verdampfungseinheit V1 wurde das zweiphasige Gemisch temperaturgeregelt bei 54,4 °C und 70 mbar abs. weiter abgedampft und anschließend in den Abscheidebehälter der Verdampfungseinheit V1 geleitet. Der Abscheidebehälter war mit einem Tropfenabscheider ausgestattet, um sicher Schwefelsäure und PTZ abzutrennen.

Die nicht gasförmigen Bestandteile im Abscheidebehälter der Verdampfungseinheit V1 wurden als erste Schwersiederphase SPh1 über einen Umpumpstrom mittels eines Solekühlers auf -2 °C gekühlt. Im Abscheidebehälter stellte sich so eine Mischtemperatur von ca. 4 bis 5 °C ein. Ein Teil des Umpumpstroms (2.235 kg/h) der ersten Schwersiederphase SPh1 wurde zur Schwefelsäurerückführung dem Reaktor R1 wieder zugeführt. Darüber hinaus wurde ein Teil des Umpumpstroms der ersten Schwersiederphase SPh1 (106 kg/h) dem Dünnschichtverdampfer V2 (4 m², Nickel-Chrom-Molybdän-Legierung 2.4610) zugeführt, um weitere Wertprodukte (TBA, AS) über den Kopf des Dünnschichtverdampfers V2 abzutrennen (70 °C, 60 mbar abs.). Die Beheizung des Dünnschichtverdampfers V2 erfolgte mittels Niederdruck-Dampf. Dem Sumpfaustrag des Dünnschichtverdampfers V2 war eine Pumpe (in Fig.1 nicht dargestellt) nachgeschaltet, welche die auszuschleusende zweite Schwersiederphase SPh2 in einem Teilstrom zum Absetzbehälter A1 leitete. Auf dem Weg zum Absetzbehälter A1 wurde der Teilstrom der zweiten Schwersiederphase SPh2 von 70 °C auf 35 °C gekühlt. Dies erfolgte mittels eines Mantelrohrs, durch das im Gegenstrom Wasser mit einer Temperatur von 30 °C geführt wurde.

Ein weiterer Teilstrom der zweiten Schwersiederphase SPh2 des Dünnschichtverdampfers V2 wurde wiederum als heißer Rückführstrom direkt dem Zufuhrstrom zum Dünnschichtverdampfer V2 zugemischt. Durch Variation des heißen Rückführstroms ließen sich der Zufuhrstrom, sowie auch die Zufuhrstromtemperatur in einem weiten Bereich einstellen. In Verbindung mit der Anpassung der Heizdampfmenge und der Heizdampftemperatur war der Dünnschichtverdampfer V2 in der Lage, einen großen Lastbereich abzudecken.

Noch ein weiterer Teilstrom der zweiten Schwersiederphase SPh2 des Dünnschichtverdampfers wurde dem kalten Umpumpstrom auf der Saugseite der Pumpe zum Dünnschichtverdampfer V2 zugegeben, der sich dadurch aber nur wenig erwärmte. Der Zufuhrstrom zum Dünnschichtverdampfer V2 wurde auf der Druckseite der Pumpe entnommen.
Der Brüden des Dünnschichtverdampfers V2 wurde über die Leitung 5 dem Abscheidebehälter der Verdampfungseinheit V1 zugeführt. Der Brüden B1 aus dem Abscheidebehälter der Verdampfungseinheit V1 (ca. 53 °C) hatte folgende Zusammensetzung:

| | |
|---|---|
| 66% | TBA |
| 22% | AS |
| 10% | Isobuten |
| 3% | sonstige Bestandteile |

Der Brüden B1 wurde fraktionierend kondensiert und dazu in den Kopf des Kondensators C1 (Rohrbündelwärmetauscher, 75 m², Kühlung: Flusswasser (27 °C), 60 mbar abs., Edelstahl 1.4571) geleitet. Im Kondensator C1 wurde das zugeführte Gemisch auf 29 °C gekühlt.

Der Brüden B2 des Kondensators C1 (umfassend ca. 56% TBA, 5% AS, 36% Isobuten) wurde in den Kopf des Kondensators C2 (Rohrbündelwärmetauscher, 30 m², Kühlung: Kühlsole (-20 °C), 60 mbar abs., Edelstahl 1.4571) geführt. Das Kondensat K2 des Kondensators C2 (umfassend ca. 84% TBA, 7% AS, 5% Isobuten, ca. -17 °C) wurde in einem Behälter (in Fig. 1 nicht dargestellt) mit dem Kondensat K1 des Kondensators C1 vereinigt. Der Brüden B3 des Kondensators C2 (umfassend ca. 95% Isobuten) wurde über eine Pumpe (in Fig. 1 nicht dargestellt) mit dem Brüden B6 des Kondensators C4 gemischt und in den Reaktor R1 zurückgeführt.

Das Kondensat K1 des Kondensators C1 (umfassend ca. 73% TBA, 24% AS, 0,5% Isobuten) wurden in einem Behälter (in Fig. 1 nicht dargestellt) mit dem Kondensat K2 des Kondensators C2 vereinigt. Das vereinigte Kondensat aus C1 und C2 hatte folgende Zusammensetzung:

| | |
|---|---|
| 72,70% | TBA |
| 23,93% | AS |
| 1,49% | Diisobuten |
| 0,62% | Isobuten |
| 1,26% | sonstige Bestandteile |

Ein Teilstrom der vereinigten Kondensate aus C1 und C2 wurde zusammen mit einer 4-Hydroxy-TEMPO (4-HT)-Lösung (2% in TBA) als Stabilisator I2 in den Kopf des Kondensators C1 geleitet, wiederum ein Teilstrom davon wurde als Stabilisator I3 in den Kopf des Kondensators C2 geleitet.

Ein weiterer Teilstrom der vereinigten Kondensate aus den Kondensatoren C1 und C2 wurde der Destillationskolonne D1 (40 Dual-Flow-Böden, 79 °C im Kolonnensumpf, 120 mbar abs. im Kolonnenkopf) auf den Boden 23 zugeführt. Die Destillationskolonne D1 wurde über einen Naturumlaufverdampfer (4 bar abs. Dampf) geheizt. Die Temperaturregelung der Destillationskolonne D1 erfolgte über ein Regelventil in der Rücklaufleitung. Das Vakuum wurde mittels Regelventil in der Saugleitung zur Vakuumeinheit geregelt.

Der Brüden B4 aus der Destillationskolonne D1 wurde fraktionierend kondensiert und dazu in den Kondensator C3 (Rohrbündelwärmetauscher, 110 m², Kühlung: Flusswasser (27 °C), 120 mbar abs., Edelstahl 1.4571) geleitet. Im Kondensator C3 wurde das zugeführte Gemisch auf 29 °C gekühlt. Das Kondensat K3 des Kondensators C3 wurde in einem Behälter mit dem Kondensat K4 des Kondensators C4 vereinigt.

Der Brüden B5 des Kondensators C3 wurde in den Kondensator C4 (Rohrbündelwärmetauscher, 8 m², Kühlung: Kühlsole (-20 °C), 120 mbar abs., Edelstahl 1.4571) geleitet und auf -2 °C gekühlt. Das Kondensat K4 des Kondensators C4 wurde in einem Behälter (in Fig. 1 nicht dargestellt) mit dem Kondensat K3 des Kondensators C3 vereinigt. Der Brüden B6 des Kondensators C4 (147,7 m³/h, 69,76% Isobuten) wurde über eine Pumpe (in Fig. 1 nicht dargestellt) mit dem Brüden B3 des Kondensators C2 und dem Kondensat des Rücklaufkühlers W1 gemischt und in den Reaktor R1 zurückgeführt.

Ein Teilstrom der vereinigten Kondensate aus C3 und C4 wurde in den Kopf der Destillationskolonne D1 geleitet; wiederum ein Teilstrom davon wurde als Gemisch mit einer 4-HT-Lösung (2% in TBA) als Stabilisator I4 in den Kopf des Kondensators C3 geleitet.

Das Sumpfprodukt der Destillationskolonne 1 hatte folgende Zusammensetzung:

| | |
|---|---|
| 74,46% | TBA |
| 24,37% | AS |
| 1,17% | sonstige Bestandteile |

Das Sumpfprodukt S1 der Destillationskolonne D1 wurde mit einer 4-HT-Lösung (2% in TBA) als Stabilisator I5 versetzt und der Destillationskolonne D2 (40 Dual-Flow-Böden, 92 °C im Kolonnensumpf, 75 mbar abs. im Kolonnenkopf) auf den Boden 18 zugeführt. Die Destillationskolonne D2 wurde über einen Naturumlaufverdampfer (4 bar abs. Dampf) geheizt. Die Temperaturregelung der Destillationskolonne D2 erfolgte über ein Regelventil in der Rücklaufleitung. Das Vakuum wurde mittels Regelventil in der Saugleitung zur Vakuumeinheit geregelt.

In den Sumpf der Destillationskolonne D2 wurden 6m³/h Magerluft (5 Vol.-% Sauerstoff in Stickstoff) dosiert.

Der Brüden B7 aus der Destillationskolonne D2 (umfassend 99,57% TBA) wurde fraktionierend kondensiert und dazu in den Kondensator C5 (Rohrbündelwärmetauscher, 72 m², Kühlung: Flusswasser (27 °C), 70 mbar abs., Edelstahl 1.4571) geführt. Im Kondensator C5 wurde das zugeführte Gemisch auf 29 °C gekühlt. Das Kondensat P1 des Kondensators C5 wurde in einem Behälter (in Fig. 1 nicht dargestellt) mit dem Kondensat P2 des Kondensators C6 vereinigt.

Der Brüden B8 des Kondensators C5 wurde in den Kopf des Kondensators C6 (Rohrbündelwärmetauscher, 12 m², Kühlung: Kühlsole (-20 °C), 65 mbar abs. Edelstahl 1.4571) geleitet und auf-17 °C gekühlt. Das Kondensat P2 des Kondensators C6 wurde in einem Behälter (in Fig. 1 nicht dargestellt) mit dem Kondensat P1 des Kondensators C5 als Produkt vereinigt. Der Brüden B9 des Kondensators C6 wurde über eine Pumpe (in Fig. 1 nicht dargestellt) aus dem Verfahren ausgetragen.

Ein Teilstrom der vereinigten Kondensate P1 und P2 aus den Kondensatoren C5 und C6 wurde unter Zusatz von 4-Methoxyphenol (MEHQ, 2% in TBA)-Lösung als Stabilisator I6 als Rücklauf in die Destillationskolonne D2 geleitet. Weitere Teilströme der vereinigten Kondensate P1 und P2 aus den Kondensatoren C5 und C6 wurden unter Zusatz von 4-Methoxyphenol (MEHQ, 2% in TBA)-Lösung als Stabilisatoren I7 und I8 den Kondensatoren C5 bzw. C6 zugeführt.

Hierbei wurde die Stabilisierung der Kolonne mit einem höheren Gehalt an MEHQ vorgenommen, während der 4-Methoxyphenol-Gehalt in den Kondensatoren C5 und C6 15 +/- 5 ppm betrug. Um die Kondensation von TBA am Kolonnenkopf der Destillationskolonne D2 zu vermeiden, welche auch zur Polymerisation von TBA führen könnte, wurde der Kolonnenkopf mit Dampf (4 bar abs.) beheizt.

Noch ein weiterer Teilstrom der vereinigten Kondensate P1 und P2 aus den Kondensatoren C5 und C6 wurde nach einer Druckerhöhung auf 4 bar abs. über einen Wärmetauscher (Spiral-Wärmetauscher, Kühlung: Kühlsole (-20 °C), in Fig.1 nicht dargestellt) auf 20 °C gekühlt und als Produkt aus dem Verfahren ausgetragen. Ein Teilstrom davon wurde als Lösungsmittel für die Stabilisatoren 4-HT und MEHQ verwendet.

Das Produkt hatte folgende Zusammensetzung:

| | |
|---|---|
| 99,83% | TBA |
| 0,05% | Isobuten |
| 0,02% | tert-Butylpropionat |
| 15 ppm | MEHQ |

Der Sumpf S2 der Destillationskolonne D2 (umfassend 93,12% AS) wurde nach einer Druckerhöhung auf 4 bar abs. über einen Wärmetauscher (Spiral-Wärmetauscher, 5 m², Kühlung: Warmwasser Edelstahl 1.4571, in Fig.1 nicht dargestellt) auf 30 ° gekühlt und ein Teilstrom mit dem Zufuhrstrom der Acrylsäure vereinigt und dem Reaktor R1 zugeführt.

Der in der Verdampfungseinheit V1 und den nachgeschalteten Einheiten benötigte Unterdruck wurde mittels einer Vakuumeinheit erzeugt. Es wurden Drehwälzkolbenverdichter ohne Schmieröl verwendet.

Zur Herstellung der Stabilisatorlösung von Phenothiazin wurde Acrylsäure rein in einem Rührbehälter (begleitbeheizt mit Wasser, 30 °C, belüftet) vorgelegt. PTZ wurde als Feststoff über eine Sackentleerstation und ein pneumatisches Powder-Transfer-System mittels Anlegen von Vakuum über eine Pumpe in den Rührbehälter eingeführt. Geringe Mengen an Magerluft (5 Vol.-% Sauerstoff in Stickstoff) wurden zugegeben. PTZ wurde unter Rühren gelöst und die PTZ-Lösung in einen Vorlagebehälter (begleitbeheizt mit Wasser, 30 °C, belüftet) geleitet, von diesem aus die Dosierung ins Verfahren vorgenommen wurde.

Zur Herstellung der Stabilisatorlösung von 4-Hydroxy-2,2,6,6-tetramethyl-1-oxylpiperidin wurde tert-Butylacrylat (aus den vereinigten Kondensaten P1 und P2 der Kondensatoren C5 und C6) in einem Rührbehälter (belüftet) vorgelegt. 4-HT wurde als Feststoff über eine Sackentleerstation und ein pneumatisches Powder-Transfer-System mittels Anlegen von Vakuum über eine Pumpe in den Rührbehälter eingeführt. Geringe Mengen an Magerluft (5 Vol.-% Sauerstoff in Stickstoff) wurden zugegeben. 4-HT wurde unter Rühren gelöst und die 4-HT-Lösung in einen Vorlagebehälter (belüftet) geleitet, von diesem aus die Dosierung ins Verfahren vorgenommen wurde.

Zur Herstellung der Stabilisatorlösung von 4-Methoxyphenol wurde tert-Butylacrylat (aus den vereinigten Kondensaten P1 und P2 der Kondensatoren C5 und C6) in einem Rührbehälter (belüftet) vorgelegt. MEHQ wurde als Feststoff über eine Sackentleerstation und ein pneumatisches Powder-Transfer-System mittels Anlegen von Vakuum über eine Pumpe in den Rührbehälter eingeführt. Geringe Mengen an Magerluft (5 Vol.-% Sauerstoff in Stickstoff) wurden zugegeben. MEHQ wurde unter Rühren gelöst und die MEHQ-Lösung in einen Vorlagebehälter (belüftet) geleitet, von diesem aus die Dosierung ins Verfahren vorgenommen wurde.

Die in den Solekühlern verwendete Sole war als Druckkreis aufgebaut. Die Sole wurde in einer Ammoniakkälteanlage auf-20 °C gekühlt und den jeweiligen Verfahrenselementen zugeführt. Danach wurde die Sole in einem Solevorlagebehälter homogenisiert und über eine Pumpe wieder der Ammoniakkälteanlage zugeführt. Das Solesystem verfügte über ein Ausgleichsgefäß, welches mit Magerluft (5 Vol.-% Sauerstoff in Stickstoff) überlagert wurde.

Beim Verfahren anfallendes, nicht verwertbares Abgas wurde über einen Abscheider geführt und die nicht kondensierten Bestandteile an einer Schirmfackel verbrannt, während das Kondensat ausgeführt wurde.

Es ist erkennbar, dass das Verfahren die Herstellung von tert-Butylacrylsäureester in hoher Reinheit (hier 99,94%) bei gleichzeitig energetisch günstiger Abtrennung von Isobuten, welches mit hohem Abtrennungsgrad aus dem Veresterungsgemisch isoliert werden konnte, erlaubt.

### Beispiel 2a

Der erste Brüden B1 wurde zweistufig (bei zwei verschiedenen Temperaturen) bzw. einstufig partiell kondensiert und die Zusammensetzung des Kondensats bzw. des nicht kondensierten Brüdens untersucht. Alle angegebenen Prozentsätze sind gewichtsbezogen, soweit nicht anders angegeben. Der Brüden B1 (ca. 53 °C) hatte folgende Zusammensetzung:

| | | |
|---|---|---|
| 1.486 kg/h | (66%) | TBA |
| 494 kg/h | (22%) | Acrylsäure |
| 218 kg/h | (10%) | Isobuten |
| 58 kg/h | (3%) | sonstige Bestandteile |

Die zweistufige Partialkondensation wurde bei 33 °C bzw. -18 °C (Temperatur der jeweiligen Kondensate) durchgeführt. Als Vergleich wurde eine einstufige Partialkondensation bei -18 °C gewählt.

Die Kühlleistung für die zweistufige Partialkondensation betrug insgesamt 255 kW (202 kW für die im Kondensator C1 durchgeführte erste partielle Kondensation und 53 kW für die im Kondensator C2 durchgeführte zweite partielle Kondensation). Die Kühlleistung für die einstufige Partialkondensation betrug 311 kW. Es ist erkennbar, dass die benötigte Kühlleistung bei einer zweistufigen Partialkondensation geringer ist als bei einer einstufigen Partialkondensation.

Die Zusammensetzungen der jeweiligen Kondensate bzw. Brüden sind in Tabelle A angegeben.

**Tabelle A**

| | T [°C] | | Volumenstrom [kg/h] | Zusammensetzung [Gew.-%] |
|---|---|---|---|---|
| | | Zweistufige Partialkondensation | | |
| Kondensat 1. Kondensator | 33,0 | Isobuten | 9 | 0,5 |
| | | Acrylsäure | 462 | 27,2 |
| | | tert-Butylacrylat | 1180 | 69,6 |
| Brüden 1. Kondensator | 33,0 | Isobuten | 209 | 37,3 |
| | | Acrylsäure | 32 | 5,7 |
| | | tert-Butylacrylat | 306 | 54,6 |
| Kondensat 2. Kondensator | -18,0 | Isobuten | 19 | 5,0 |
| | | Acrylsäure | 32 | 8,3 |
| | | tert-Butylacrylat | 321 | 83,6 |
| | | sonstige Bestandteile | 12 | 3,1 |
| Brüden 2. Kondensator | -18,0 | Isobuten | 190 | 96,0 |
| | | Acrylsäure | 0 | 0,0 |
| | | tert-Butylacrylat | 6 | 3,0 |
| Vereinigte Kondensate | 23,9 | Isobuten | 28 | 1,4 |
| | | Acrylsäure | 494 | 23,7 |
| | | tert-Butylacrylat | 1500 | 72,2 |

| | | Einstufige Partialkondensation | | |
|---|---|---|---|---|
| Brüden | -18,0 | Isobuten | 97 | 96,7 |
| | | Acrylsäure | 0 | 0,1 |
| | | tert-Butylacrylat | 2 | 2,4 |
| Kondensat | -18,0 | Isobuten | 121 | 5,6 |
| | | Acrylsäure | 493 | 22,9 |
| | | tert-Butylacrylat | 1483 | 68,8 |

Es ist erkennbar, dass die vereinigten Kondensate der zweistufigen Partialkondensation einen niedrigeren Isobuten-Anteil enthielten als das Kondensat der einstufigen Partialkondensation. Dementsprechend mehr Isobuten war im Brüden des 2. Kondensators der zweistufigen Partialkondensation enthalten als im Brüden der einstufigen Partialkondensation. Durch die zweistufige Partialkondensation bei einer ersten Temperatur und einer zweiten tieferen Temperatur wird insgesamt eine höhere Trennschärfe erreicht als durch eine einstufige Partialkondensation bei der zweiten Temperatur.

Es ist außerdem erkennbar, dass die Temperatur der vereinigten Kondensate der zweistufigen Partialkondensation höher war als die Temperatur des Kondensats der einstufigen Partialkondensation.

### Beispiel 2b

Der vierte Brüden B4 wurde zweistufig (bei zwei verschiedenen Temperaturen) bzw. einstufig partiell kondensiert und die Zusammensetzung des Kondensats bzw. des nicht kondensierten Brüdens untersucht. Alle angegebenen Prozentsätze sind gewichtsbezogen, soweit nicht anders angegeben. Der Brüden B4 (ca. 37 °C) hatte folgende Zusammensetzung:

| | | |
|---|---|---|
| 55 kg/h | (5,3%) | Isobuten |
| 18 kg/h | (1,8%) | Wasser |
| 49 kg/h | (4,8%) | tert-Butylacrylat |
| 41 kg/h | (4,0%) | tert-Butylacetat |
| 744 kg/h | (72,5%) | Diisobuten |
| 114 kg/h | (11,1%) | tert-Butanol |

Die zweistufige Partialkondensation wurde bei 33 °C bzw. -18 °C (Temperatur der jeweiligen Kondensate) durchgeführt. Als Vergleich wurde eine einstufige Partialkondensation bei -18 °C gewählt.

Die Kühlleistung für die zweistufige Partialkondensation betrug insgesamt 126 kW (50 kW für die im Kondensator C3 durchgeführte erste partielle Kondensation und 76 kW für die im Kondensator C2 durchgeführte zweite partielle Kondensation). Die Kühlleistung für die einstufige Partialkondensation betrug 142 kW. Es ist erkennbar, dass die benötigte Kühlleistung bei einer zweistufigen Partialkondensation geringer ist als bei einer einstufigen Partialkondensation.

Die Zusammensetzungen der jeweiligen Kondensate bzw. Brüden sind in Tabelle B angegeben.

**Tabelle B**

| | | | | |
|---|---|---|---|---|
| | T [°C] | | Volumenstrom [kg/h] | Zusammensetzung [Gew.-%] |

| | | Zweistufige Partialkondensation | | |
|---|---|---|---|---|
| Kondensat 1. Kondensator | 33,0 | Isobuten | 1 | 0,2 |
| | | tert-Butylacrylat | 43 | 8,6 |
| | | tert-Butylacetat | 23 | 4,7 |
| | | Diisobuten | 376 | 74,6 |
| | | tert-Butanol | 59 | 11,7 |
| Brüden 1. Kondensator | 33,0 | Isobuten | 53 | 10,0 |
| | | tert-Butylacrylat | 17 | 3,11 |
| | | tert-Butylacetat | 18 | 3,4 |
| | | Diisobuten | 367 | 69,0 |
| | | tert-Butanol | 55 | 10,3 |
| Kondensat 2. Kondensator | -18,0 | Isobuten | 42 | 8,1 |
| | | tert-Butylacrylat | 17 | 3,2 |
| | | tert-Butylacetat | 18 | 3,5 |
| | | Diisobuten | 367 | 71,1 |
| | | tert-Butanol | 55 | 10,7 |
| Brüden 2. Kondensator | -18,0 | Isobuten | 12 | 66,2 |
| | | tert-Butylacrylat | 0 | 0,1 |
| | | tert-Butylacetat | 0 | 0,1 |
| | | Diisobuten | 1 | 7,2 |
| | | tert-Butanol | 0 | 0,0 |
| Vereinigte Kondensate | 7,5 | Isobuten | 43 | 4,2 |
| | | tert-Butylacrylat | 60 | 5,9 |
| | | tert-Butylacetat | 41 | 4,1 |
| | | Diisobuten | 742 | 72,9 |
| | | tert-Butanol | 114 | 11,2 |

| | | Einstufige Partialkondensation | | |
|---|---|---|---|---|
| Brüden | -18,0 | Isobuten | 5 | 48,7 |
| | | tert-Butylacrylat | 0 | 0,2 |
| | | tert-Butylacetat | 0 | 0,1 |
| | | Diisobuten | 1 | 8,2 |
| | | tert-Butanol | 0 | 0,0 |
| Kondensat | -18,0 | Isobuten | 49 | 4,8 |
| | | tert-Butylacrylat | 60 | 5,8 |
| | | tert-Butylacetat | 41 | 4,0 |
| | | Diisobuten | 743 | 72,4 |
| | | tert-Butanol | 114 | 11,1 |

Es ist erkennbar, dass die vereinigten Kondensate der zweistufigen Partialkondensation einen niedrigeren Isobuten-Anteil enthielten als das Kondensat der einstufigen Partialkondensation. Die zweistufige Partialkondensation erlaubt einen geringeren Isobuten-Verlust über die Leichtsiederausschleusung gegenüber der einstufigen Partialkondensation.

## Patentansprüche

1. Verfahren zur kontinuierlichen Herstellung des tert-Butylesters einer aliphatischen C₁-C₄-Carbonsäure, bei dem man:
a) eine aliphatische C₁-C₄-Carbonsäure mit Isobuten in Gegenwart eines sauren Katalysators zu einem Veresterungsgemisch (G1) umsetzt;
b) das Veresterungsgemisch (G1) partiell verdampft, wodurch man eine den sauren Katalysator enthaltende flüssige erste Schwersiederphase (SPh1), und einen tert-Butylester enthaltenden ersten Brüden (B1) erhält;
c) den ersten Brüden (B1) fraktionierend kondensiert, indem man den ersten Brüden (B1) bei einem ersten Druck und einer ersten Temperatur partiell kondensiert und ein erstes Kondensat (K1) erhält, den nicht kondensierten zweiten Brüden (B2) bei einem zweiten Druck und einer zweiten Temperatur partiell kondensiert und ein zweites Kondensat (K2) erhält, wobei die erste Temperatur 0 bis 45 °C unterhalb der Kondensationstemperatur des tert-Butylesters beim ersten Druck liegt und die zweite Temperatur 45 bis 80 °C unterhalb der Kondensationstemperatur des tert-Butylesters beim zweiten Druck liegt, mit der Maßgabe, dass die zweite Temperatur mindestens 5 °C unterhalb der ersten Temperatur liegt; und
d) das erste Kondensat (K1) und das zweite Kondensat (K2) vereinigt und einer gemeinsamen Aufarbeitung zuführt, und den bei der zweiten Temperatur nicht kondensierten dritten Brüden (B3) in den Schritt a) zurückführt.

2. Verfahren nach Anspruch 1, bei dem man das vereinigte Kondensat einer ersten Destillationskolonne (D1) zuführt, wobei man ein erstes flüssiges Sumpfprodukt (S1) und einen vierten Brüden (B4) erhält; das erste flüssige Sumpfprodukt (S1) einer weiteren Aufarbeitung zuführt; den vierten Brüden (B4) fraktionierend kondensiert, indem man den vierten Brüden (B4) bei einem dritten Druck und einer dritten Temperatur partiell kondensiert und ein erstes Leichtsiederkondensat (K3) erhält, den nicht kondensierten fünften Brüden (B5) bei einem vierten Druck und einer vierten Temperatur partiell kondensiert und ein zweites Leichtsiederkondensat (K4) erhält, wobei die vierte Temperatur niedriger ist als die dritte Temperatur; man den bei der vierten Temperatur nicht kondensierten sechsten Brüden (B6) in den Schritt a) zurückführt; und das erste und/oder das zweite Leichtsiederkondensat (K3) und (K4) teilweise als Rücklauf zum Kopf der ersten Destillationskolonne (D1) zurückführt.

3. Verfahren nach Anspruch 2, wobei die dritte Temperatur 5 bis 40 °C unterhalb der Kondensationstemperatur von Diisobuten beim dritten Druck liegt und die vierte Temperatur 30 bis 55 °C unterhalb der Kondensationstemperatur von Diisobuten beim vierten Druck liegt, mit der Maßgabe, dass die vierte Temperatur mindestens 5 °C unterhalb der dritten Temperatur liegt.

4. Verfahren nach einem der Ansprüche 2 oder 3, bei dem man das erste flüssige Sumpfprodukt (S1) einer zweiten Destillationskolonne (D2) zuführt, wobei man ein zweites flüssiges Sumpfprodukt (S2) und einen siebten Brüden (B7) erhält; das zweite flüssige Sumpfprodukt (S2) zumindest teilweise in den Schritt a) zurückführt; den siebten Brüden (B7) fraktionierend kondensiert, indem man den siebten Brüden (B7) bei einem fünften Druck und einer fünften Temperatur partiell kondensiert und ein erstes Produktkondensat (P1) erhält, den nicht kondensierten achten Brüden (B8) bei einem sechsten Druck und einer sechsten Temperatur partiell kondensiert und ein zweites Produktkondensat (P2) erhält, wobei die sechste Temperatur niedriger ist als die fünfte Temperatur; und das erste und/oder das zweite Produktkondensat (P1) und (P2) teilweise als Rücklauf in die zweite Destillationskolonne (D2) zurückführt.

5. Verfahren nach Anspruch 4, wobei die fünfte Temperatur 0 bis 45 °C unterhalb der Kondensationstemperatur des tert-Butylesters beim fünften Druck liegt und die sechste Temperatur 45 bis 80 °C unterhalb der Kondensationstemperatur des tert-Butylesters beim sechsten Druck liegt, mit der Maßgabe, dass die sechste Temperatur mindestens 5 °C unterhalb der fünften Temperatur liegt.

6. Verfahren nach einem der vorherigen Ansprüche, wobei der erste Druck im Bereich von 10 bis 200 mbar abs. liegt.

7. Verfahren nach einem der vorherigen Ansprüche, wobei der zweite Druck im Bereich von 10 bis 200 mbar abs. liegt.

8. Verfahren nach einem der vorherigen Ansprüche, wobei die aliphatische C₁-C₄-Carbonsäure ausgewählt ist unter Acrylsäure und Methacrylsäure.

9. Verfahren nach einem der vorherigen Ansprüche, wobei das Veresterungsgemisch (G1) 0,5 bis 5,0 Gew.-% des sauren Katalysators umfasst.

10. Verfahren nach einem der vorherigen Ansprüche, wobei der saure Katalysator eine anorganische Säure oder eine organische Säure ist.

11. Verfahren nach einem der vorhergehenden Ansprüche, wobei die Umsetzung in Schritt a) in Gegenwart eines unter Phenothiazinen ausgewählten Stabilisators durchgeführt wird.

12. Verfahren nach einem der vorhergehenden Ansprüche, wobei die fraktionierende Kondensation c) in Gegenwart eines unter N-Oxyl-Verbindungen ausgewählten Stabilisators durchgeführt wird.

13. Verfahren nach einem der Ansprüche 2 bis 12, wobei die fraktionierende Kondensation des vierten Brüdens (B4) in Gegenwart eines unter N-Oxyl-Verbindungen ausgewählten Stabilisators durchgeführt wird.

14. Verfahren nach einem der Ansprüche 2 bis 13, wobei dem ersten flüssigen Sumpfprodukt (S1) ein unter N-Oxyl-Verbindungen ausgewählter Stabilisator zugesetzt wird.

15. Verfahren nach einem der Ansprüche 4 bis 14, wobei in den Verstärkungsteil der zweiten Destillationskolonne (D2) ein unter Phenolverbindungen ausgewählter Stabilisator dosiert wird.

## Claims

1. A process for continuously preparing the tert-butyl ester of an aliphatic C₁-C₄ carboxylic acid, in which:
a) an aliphatic C₁-C₄ carboxylic acid is reacted with isobutene in the presence of an acidic catalyst to give an esterification mixture (G1);
b) the esterification mixture (G1) is partially evaporated, giving a liquid first high boiler phase (SPh1) comprising the acidic catalyst, and a first vapor (B1) comprising tert-butyl ester;
c) the first vapor (B1) is fractionally condensed by partially condensing the first vapor (B1) at a first pressure and a first temperature and obtaining a first condensate (K1), partially condensing the uncondensed second vapor (B2) at a second pressure and a second temperature and obtaining a second condensate (K2), the first temperature being 0 to 45°C below the condensation temperature of the tert-butyl ester at the first pressure and the second temperature being 45 to 80°C below the condensation temperature of the tert-butyl ester at the second pressure, with the proviso that the second temperature is at least 5°C below the first temperature; and
d) the first condensate (K1) and the second condensate (K2) are combined and fed to a combined workup, and the third vapor (B3) not condensed at the second temperature is recycled into step a).

2. The process according to claim 1, in which the combined condensate is fed to a first distillation column (D1) wherein a first liquid bottom product (S1) and a fourth vapor (B4) are obtained; the first liquid bottom product (S1) is fed to a further workup; the fourth vapor (B4) is fractionally condensed by partially condensing the fourth vapor (B4) at a third pressure and a third temperature and obtaining a first low boiler condensate (K3), the uncondensed fifth vapor (B5) is partially condensed at a fourth pressure and a fourth temperature and a second low boiler condensate (K4) is obtained, the fourth temperature being lower than the third temperature; the sixth vapor (B6) uncondensed at the fourth temperature is recycled into step a); and the first and/or second low boiler condensate (K3) and (K4) is recycled partly as reflux to the top of the first distillation column (D1).

3. The process according to claim 2, wherein the third temperature is 5 to 40°C below the condensation temperature of diisobutene at the third pressure and the fourth temperature is 30 to 55°C below the condensation temperature of diisobutene at the fourth pressure, with the proviso that the fourth temperature is at least 5°C below the third temperature.

4. The process according to either of claims 2 and 3, in which the first liquid bottom product (S1) is fed to a second distillation column (D2) to obtain a second liquid bottom product (S2) and a seventh vapor (B7); the second liquid bottom product (S2) is at least partly recycled into step a); the seventh vapor (B7) is fractionally condensed by partially condensing the seventh vapor (B7) at a fifth pressure and a fifth temperature and obtaining a first product condensate (P1), the uncondensed eighth vapor (B8) is partially condensed at a sixth pressure and a sixth temperature and a second product condensate (P2) is obtained, the sixth temperature being lower than the fifth temperature; and the first and/or second product condensate (P1) and (P2) is recycled partly as reflux into the second distillation column (D2).

5. The process according to claim 4, wherein the fifth temperature is 0 to 45°C below the condensation temperature of the tert-butyl ester at the fifth pressure and the sixth temperature is 45 to 80°C below the condensation temperature of the tert-butyl ester at the sixth pressure, with the proviso that the sixth temperature is at least 5°C below the fifth temperature.

6. The process according to any of the preceding claims, wherein the first pressure is in the range from 10 to 200 mbar abs.

7. The process according to any of the preceding claims, wherein the second pressure is in the range from 10 to 200 mbar abs.

8. The process according to any of the preceding claims, wherein the aliphatic C₁-C₄ carboxylic acid is selected from acrylic acid and methacrylic acid.

9. The process according to any of the preceding claims, wherein the esterification mixture (G1) comprises 0.5% to 5.0% by weight of the acidic catalyst.

10. The process according to any of the preceding claims, wherein the acidic catalyst is an inorganic acid or an organic acid.

11. The process according to any of the preceding claims, wherein the reaction in step a) is conducted in the presence of a stabilizer selected from phenothiazines.

12. The process according to any of the preceding claims, wherein the fractional condensation c) is conducted in the presence of a stabilizer selected from N-oxyl compounds.

13. The process according to any of claims 2 to 12, wherein the fractional condensation of the fourth vapor (B4) is conducted in the presence of a stabilizer selected from N-oxyl compounds.

14. The process according to any of claims 2 to 13, wherein a stabilizer selected from N-oxyl compounds is added to the first liquid bottom product (S1).

15. The process according to any of claims 4 to 14, wherein a stabilizer selected from phenol compounds is metered into the rectifying section of the second distillation column (D2).

## Revendications

1. Procédé de fabrication continue de l'ester tert-butylique d'un acide carboxylique en C₁-C₄ aliphatique, selon lequel :
a) un acide carboxylique en C₁-C₄ aliphatique est mis en réaction avec de l'isobutène en présence d'un catalyseur acide pour former un mélange d'estérification (G1) ;
b) le mélange d'estérification (G1) est partiellement évaporé, une première phase liquide de composants de point d'ébullition élevé contenant le catalyseur acide (SPh1) et des premières vapeurs contenant l'ester tert-butylique (B1) étant obtenues ;
c) les premières vapeurs (B1) sont soumises à une condensation fractionnée, par condensation partielle des premières vapeurs (B1) à une première pression et une première température et obtention d'un premier condensat (K1), condensation partielle des deuxièmes vapeurs non condensées (B2) à une deuxième pression et une deuxième température et obtention d'un deuxième condensat (K2), la première température étant située 0 à 45 °C en dessous de la température de condensation de l'ester tert-butylique à la première pression et la deuxième température étant située 45 à 80 °C en dessous de la température de condensation de l'ester tert-butylique à la deuxième pression, à condition que la deuxième température soit située au moins 5 °C en dessous de la première température ; et
d) le premier condensat (K1) et le deuxième condensat (K2) sont réunis et introduits dans un traitement commun, et les troisièmes vapeurs (B3) non condensées à la deuxième température sont recyclées dans l'étape a).

2. Procédé selon la revendication 1, selon lequel le condensat réuni est introduit dans une première colonne de distillation (D1), un premier produit de fond liquide (S1) et des quatrièmes vapeurs (B4) étant obtenus ; le premier produit de fond liquide (S1) étant introduit dans un traitement supplémentaire ; les quatrièmes vapeurs (B4) étant soumises à une condensation fractionnée par condensation partielle des quatrièmes vapeurs (B4) à une troisième pression et une troisième température et obtention d'un premier condensat de composants de point d'ébullition faible (K3), condensation partielle des cinquièmes vapeurs non condensées (B5) à une quatrième pression et une quatrième température et obtention d'un deuxième condensat de composants de point d'ébullition faible (K4), la quatrième température étant inférieure à la troisième température ; les sixièmes vapeurs non condensées à la quatrième température (B6) étant recyclées dans l'étape a) ; et le premier et/ou le deuxième condensat de composants de point d'ébullition faible (K3) et (K4) étant partiellement recyclés en tant que reflux à la tête de la première colonne de distillation (D1).

3. Procédé selon la revendication 2, selon lequel la troisième température est située 5 à 40 °C en dessous de la température de condensation du diisobutène à la troisième pression, et la quatrième température est située 30 à 55 °C en dessous de la température de condensation du diisobutène à la quatrième pression, à condition que la quatrième température soit située au moins 5 °C en dessous de la troisième température.

4. Procédé selon l'une quelconque des revendications 2 ou 3, selon lequel le premier produit de fond liquide (S1) est introduit dans une deuxième colonne de distillation (D2), un deuxième produit de fond liquide (S2) et des septièmes vapeurs (B7) étant obtenus ; le deuxième produit de fond liquide (S2) étant au moins partiellement recyclé dans l'étape a) ; les septièmes vapeurs (B7) étant soumises à une condensation fractionnée par condensation partielle des septièmes vapeurs (B7) à une cinquième pression et une cinquième température et obtention d'un premier condensat de produit (P1), condensation partielle des huitièmes vapeurs non condensées (B8) à une sixième pression et une sixième température et obtention d'un deuxième condensat de produit (P2), la sixième température étant inférieure à la cinquième température ; et le premier et/ou le deuxième condensat de produit (P1) et (P2) étant partiellement recyclés en tant que reflux dans la deuxième colonne de distillation (D2).

5. Procédé selon la revendication 4, selon lequel la cinquième température est située 0 à 45 °C en dessous de la température de condensation de l'ester tert-butylique à la cinquième pression, et la sixième température est située 45 à 80 °C en dessous de la température de condensation de l'ester tert-butylique à la sixième pression, à condition que la sixième température soit située au moins 5 °C en dessous de la cinquième température.

6. Procédé selon l'une quelconque des revendications précédentes, selon lequel la première pression se situe dans la plage allant de 10 à 200 mbar abs.

7. Procédé selon l'une quelconque des revendications précédentes, selon lequel la deuxième pression se situe dans la plage allant de 10 à 200 mbar abs.

8. Procédé selon l'une quelconque des revendications précédentes, selon lequel l'acide carboxylique en C₁-C₄ aliphatique est choisi parmi l'acide acrylique et l'acide méthacrylique.

9. Procédé selon l'une quelconque des revendications précédentes, selon lequel le mélange d'estérification (G1) comprend 0,5 à 5,0 % en poids du catalyseur acide.

10. Procédé selon l'une quelconque des revendications précédentes, selon lequel le catalyseur acide est un acide inorganique ou un acide organique.

11. Procédé selon l'une quelconque des revendications précédentes, selon lequel la réaction à l'étape a) est réalisée en présence d'un stabilisateur choisi parmi les phénothiazines.

12. Procédé selon l'une quelconque des revendications précédentes, selon lequel la condensation fractionnée c) est réalisée en présence d'un stabilisateur choisi parmi les composés de N-oxyle.

13. Procédé selon l'une quelconque des revendications 2 à 12, selon lequel la condensation fractionnée des quatrièmes vapeurs (B4) est réalisée en présence d'un stabilisateur choisi parmi les composés de N-oxyle.

14. Procédé selon l'une quelconque des revendications 2 à 13, selon lequel un stabilisateur choisi parmi les composés de N-oxyle est ajouté au premier produit de fond liquide (S1).

15. Procédé selon l'une quelconque des revendications 4 à 14, selon lequel un stabilisateur choisi parmi les composés de phénol est ajouté dans la partie d'enrichissement de la deuxième colonne de distillation (D2) .
